(19) Europäisches Patentamt
European Patent Office
Office européen des brevets

(11) Veröffentlichungsnummer : **0 298 915 B1**

# (12) EUROPÄISCHE PATENTSCHRIFT

(45) Veröffentlichungstag der Patentschrift : **20.05.92 Patentblatt 92/21**

(51) Int. Cl.[5] : **C07D 213/70,** C07D 213/71, A01N 47/30, A01N 47/40, A01N 47/42

(21) Anmeldenummer : **88810446.0**

(22) Anmeldetag : **28.06.88**

## (54) Pyridylthio-, Pyridylsulfinyl- und Pyridylsulfonyl-phenyl-thioharnstoffe, -isothioharnstoffe und -carbodiimide, ihreHerstellung und Verwendung bei der Kontrolle von Schädlingen.

(30) Priorität : **07.07.87 CH 2578/87**
**05.05.88 CH 1689/88**

(43) Veröffentlichungstag der Anmeldung : **11.01.89 Patentblatt 89/02**

(45) Bekanntmachung des Hinweises auf die Patenterteilung : **20.05.92 Patentblatt 92/21**

(84) Benannte Vertragsstaaten : **BE CH DE FR GB IT LI NL**

(56) Entgegenhaltungen :
**DE-A- 2 501 648**
**US-A- 3 674 795**

(73) Patentinhaber : **CIBA-GEIGY AG**
**Klybeckstrasse 141**
**CH-4002 Basel (CH)**

(72) Erfinder : **Böger, Manfred**
**Wilhelm Glockstrasse 14**
**W-7858 Weil am Rhein 5 (DE)**
Erfinder : **Drabek, Jozef, Dr.**
**Benkenstrasse 12**
**CH-4104 Oberwil (CH)**
Erfinder : **Ehrenfreund, Josef, Dr.**
**Amselstrasse 11**
**CH-4123 Allschwil (CH)**

EP 0 298 915 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte Pyridylthio-, Pyridylsulfinyl- und Pyridylsulfonyl-phenyl-thioharnstoffe, -isothioharnstoffe und -carbodiimide, ihre Salze mit organischen oder anorganischen Säuren, Verfahren und Zwischenprodukte zu ihrer Herstellung, Schädlingsbekämpfungsmittel, die diese Verbindungen enthalten, sowie ihre Verwendung in der Schädlingsbekämpfung.

Die erfindungsgemässen Verbindungen entsprechen der Formel I

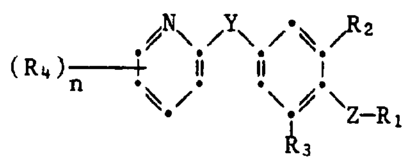

(I),

worin

$R_1$ für $C_1$-$C_{12}$-Alkyl, ein- oder mehrfach durch Halogen und/oder $C_1$-$C_6$-Alkoxy substituiertes $C_1$-$C_{12}$-Alkyl, $C_3$-$C_8$-Cycloalkyl, ein- oder mehrfach durch $C_1$-$C_3$-Alkyl substituiertes $C_3$-$C_8$-Cycloalkyl oder $C_3$-$C_8$-Cycloalkyl-$C_1$-$C_4$-alkyl;

$R_2$ und $R_3$ je für $C_1$-$C_5$-Alkyl oder $C_5$-$C_6$-Cycloalkyl;

$R_4$ je für Wasserstoff, Halogen, $C_1$-$C_4$-Alkyl oder ein- oder mehrfach durch Halogen substituiertes $C_1$-$C_4$-Alkyl;

n für 1,2,3 oder 4;

Y für S, SO oder $SO_2$;

Z für -NH-CS-NH-, -N=C($SR_5$)-NH- oder -N=C=N-; und

$R_5$ für $C_1$-$C_{10}$-Alkyl oder Allyl

stehen.

Bei den als Substituenten in Betracht kommenden Halogenen handelt es sich sowohl um Fluor und Chlor als auch um Brom und Jod, wobei Fluor und Chlor bevorzugt sind.

Die als Substituenten in Betracht kommenden Alkyle können geradkettig oder verzweigt sein. Als Beispiele solcher Alkyle seien Methyl, Aethyl, Propyl, Isopropyl, Butyl, i-Butyl, sek.Butyl, tert.Butyl oder Pentyl, Hexyl, Octyl und ihre Isomeren genannt.

Die als Substituenten in Betracht kommenden ein- oder mehrfach durch Halogen und/oder $C_1$-$C_6$-Alkoxy substituierten $C_1$-$C_{12}$-Alkyle können geradkettig oder verzweigt und nur teilweise oder auch perhalogeniert und/oder ein- bis fünffach durch $C_1$-$C_6$-Alkoxy substituiert sein, wobei für die Halogene und die Alkyle die oben gegebenen Definitionen gelten. Geeignete Beispiele solcher Substituenten sind u.a. das ein- bis dreifach durch Fluor, Chlor und/oder Brom substituierte Methyl wie z.B. $CHF_2$ oder $CF_3$; das ein- bis fünffach durch Fluor, Chlor und/oder Brom substituierte Aethyl wie z.B. $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; das ein- bis siebenfach durch Fluor, Chlor und/oder Brom substituierte Propyl oder Isopropyl wie z.B $CH_2CHBrCH_2Br$, $CF_2CHFCF_3$, $CH_2CF_2CF_3$ oder $CH(CF_3)_2$; das ein- bis neunfach durch Fluor, Chlor und/oder Brom substituierte Butyl oder eines seiner Isomeren wie z.B. $CF(CF_3)CHFCF_3$ oder $CH_2(CF_2)_2CF_3$; Methoxymethyl, Methoxyäthyl, Aethoxyäthyl, Methoxypropyl, Aethoxypropyl, Propoxypropyl, Methoxybutyl, Aethoxybutyl, Propoxybutyl oder Butoxybutyl, 1,2-Dimethoxyäthyl, 1,3-Dimethoxypropyl oder 2,4-Dimethoxybutyl. Diese Ausführungen gelten sinngemäss auch für die gegebenenfalls ein- oder mehrfach halogenierten $C_1$-$C_4$-Alkyle.

Bei den als Substituenten in Betracht kommenden Cycloalkylen handelt es sich beispielsweise um Cyclopropyl, Cyclobutyl, Cyclopentyl oder Cyclohexyl. Diese können ein- oder mehrfach durch einen $C_1$-$C_3$-Alkylrest substituiert und/oder über eine $C_1$-$C_4$-Alkylenbrücke mit dem Rest des Moleküls verbunden sein.

Die Verbindungen der Formel I, in denen Z für -N=C($SR_5$)-NH- steht, können auch in Form von Säureadditionssalzen vorliegen. Zur Bildung solcher Salze eignen sich sowohl organische als auch anorganische Säuren. Beispiele solcher Säuren sind u.a. Chlorwasserstoffsäure, Bromwasserstoffsäure, Jodwasserstoffsäure, Salpetersäure, verschiedene Phosphorsäuren, Schwefelsäure, Essigsäure, Propionsäure, Buttersäure, Valeriansäure, Oxalsäure, Malonsäure, Bernsteinsäure, Aepfelsäure, Maleinsäure, Fumarsäure, Milchsäure, Weinsäure, Zitronensäure, Benzoesäure, Phthalsäure, Zimtsäure, Phenylsulfonsäuren oder Salicylsäure.

Verbindungen der Formel I, in denen Z für -N=C($SR_5$)-NH- steht, können in den tautomeren Formen

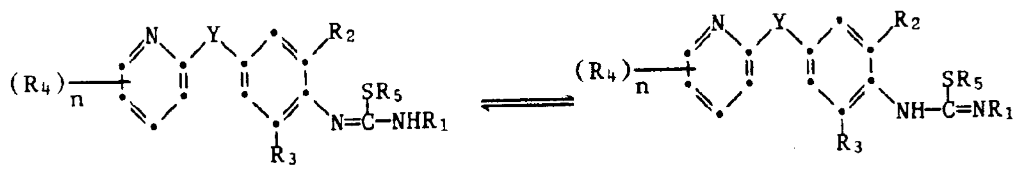

vorliegen. Die Erfindung umfasst sowohl die einzelnen Tautomeren, als auch Tautomerengemische.

Der Pyridylrest kann, je nach der Grösse von n, mehrfach durch $R_4$ substituiert sein. Ist n grösser als 1, so können die verschiedenen $R_4$ gleiche oder verschiedene Bedeutung haben.

Unter den Verbindungen der Formel I stehen diejenigen im Vordergrund, in denen $R_1$ für $C_1$-$C_8$-Alkyl, ein- oder mehrfach durch Halogen und/oder $C_1$-$C_5$-Alkoxy substituiertes $C_1$-$C_8$-Alkyl oder $C_5$-$C_6$-Cycloalkyl; $R_2$ für $C_1$-$C_5$-Alkyl; $R_3$ fur $C_1$-$C_5$-Alkyl oder Cyclopentyl; $R_4$ je für Wasserstoff oder Halogen; n für 1 oder 2; Y für S, SO oder $SO_2$; Z für -NH-CS-NH-, -N=C($SR_5$)-NH- oder -N=C=N-; und $R_5$ für $C_1$-$C_5$-Alkyl oder Allyl stehen.

Dabei sind diejenigen Verbindungen der Formel I bevorzugt, in denen

a) $R_1$ für $C_1$-$C_5$-Alkyl oder Cyclopentyl; $R_2$ und $R_3$ je für $C_1$-$C_3$-Alkyl; $R_4$; für Chlor; n für 1 oder 2; Y für S; und Z für -NH-CS-NH- stehen oder

b) $R_1$ für $C_1$-$C_5$-Alkyl oder Cyclopentyl; $R_2$ und $R_3$ je für $C_1$-$C_3$-Alkyl; $R_4$ für Chlor; n für 1 oder 2; Y für S; Z für -N=C($SR_5$)-NH-; und $R_5$ für $C_1$-$C_4$-Alkyl stehen oder

c) $R_1$ für $C_1$-$C_5$-Alkyl oder Cyclopentyl; $R_2$ und $R_3$ je für $C_1$-$C_3$-Alkyl; $R_4$ für Chlor; n für 1 oder 2; Y für S; und Z für -N=C=N- stehen.

Die erfindungsgemässen Verbindungen der Formel I können nach im Prinzip bekannten Verfahren hergestellt werden, indem man z.B.

A) ein Isothiocyanat der Formel II

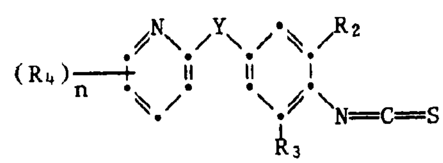

(II),

mit einem Amin der Formel III

$$H_2N—R_1 \quad (III)$$

zum Thioharnstoff umsetzt und gegebenenfalls

B) den erhaltenen Thioharnstoff mit einer Verbindung der Formel IV

$$X—R_5 \quad (IV)$$

zum Isothioharnstoff umsetzt oder

C) den erhaltenen Thioharnstoff durch Abspalten von Schwefelwasserstoff in das Carbodiimid überführt. $R_1$, $R_2$, $R_3$, $R_4$, $R_5$, Y and n haben dabei die angegebene Bedeutung und X steht für eine geeignete Abgangsgruppe wie z.B. ein Halogenatom, insbesondere Chlor, Brom oder Jod, oder Alkylsulfat.

Das Verfahren A wird üblicherweise unter normalen Druck, und in Gegenwart eines organischen Lösungs- oder Verdünnungsmittels durchgeführt. Die Temperatur beträgt dabei zwischen 0 und +150°C, vorzugsweise +10 bis 70°C. Als Lösungs- oder Verdünnungsmittel eignen sich z.B. Aether und ätherartige Verbindungen, wie Diäthyläther, Dipropyläther, Dibutyläther, Dioxan, Dimethoxyäthan oder Tetrahydrofuran; N,N-dialkylierte Carbonsäureamide aliphatische, aromatische sowie halogenierte Kohlenwasserstoffe, wie Benzol, Toluol, Xylole, Chloroform, Methylenchlorid, Tetrachlorkohlenstoff oder Chlorbenzol; Nitrile, wie Acetonitril oder Propionitril; Ketone, wie Aceton, Methyläthylketon, Methylisopropylketon, Methylisobutylketon oder Cyclohexanon.

Das Verfahren B wird zweckmässigerweise in einem inerten organischen Lösungsmittel und unter leicht erhöhtem oder normalem Druck durchgeführt. Die Temperatur beträgt dabei zwischen +10 und 250°C, vorzugsweise Siedetemperatur des verwendeten Lösungsmittels oder +50 bis 150°C. Geeignete Lösungs- oder Verdünnungsmittel sind z.B. Aether oder ätherartige Verbindungen, wie Diäthyläther, Diisopropyläther, Dioxan oder Tetrahydrofuran; aromatische Kohlenwasserstoffe, wie Benzol, Toluol oder Xylole; Ketone, wie Aceton, Methyläthylketon oder Cyclohexanon, Alkohole oder Dimethylformamid.

Das Verfahren C wird zweckmässigerweise in einem aprotischen organischen Lösungs- oder Verdünnungsmittel unter normalem Druck durchgeführt. Die Temperatur beträgt dabei zwischen 0 und +150°C, vorzugsweise +10 bis 50°C. Geeignete Lösungs- oder Verdünnungsmittel sind z.B. Aether oder ätherartige Verbindungen, wie Diäthyläther, Dipropyläther, Dibutyläther, Dioxan, Dimethoxyäthan oder Tetrahydrofuran; N,N-dialkylierte Carbonsäureamide, aliphatische, aromatische sowie halogenierte Kohlenwasserstoffe, wie Benzol, Toluol, Xylole, Chloroform, Methylenchlorid, Tetrachlorkohlenstoff oder Chlorbenzol; Nitrile, wie Acetonitril oder Propionitril; Ketone, wie Aceton, Methyläthylketon, Methylisopropylketon, oder Cyclohexanon. Die Abspaltung von Schwefelwasserstoff erfolgt nach in der Literatur beschriebenen Arbeitsweisen (T. Shibanuma, Chemistry Letters 1977, p. 575-76; S. Kim, Tetrahedron Letters 1985, p. 1661-64; W. Weith, B. 6, 1873, p. 1398; G. Amiard, Bull. Soc. chim. 1956, p. 1360). Als Abspaltungs-Reagenzien werden dabei u.a. HgO, bestimmte Pyridinium Salze, Chloressigsäureester, Cyanursäurechlorid, p-Toluolsulfochlorid oder bestimmte Phosphorsäureester-Derivate verwendet.

Die Isothiocyanate der Formel II können nach im Prinzip bekannten Methoden hergestellt werden, z.B. indem man ein Anilin der Formel V

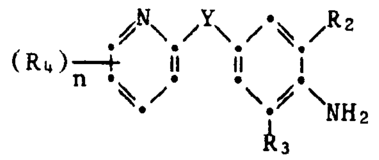 (V),

mit Thiophosgen umsetzt, wobei $R_2$, $R_3$, $R_4$ Y und n die für Formel I angegebene Bedeutung haben.

Das Verfahren zur Herstellung der Verbindungen der Formel II wird zweckmässigerweise in Gegenwart einer organischen oder anorganischen Base, wie z.B. Triäthylamin oder Calciumcarbonat und einem gegenüber den Reaktionsteilnehmern inerten Lösungs- oder Verdünnungsmittels, bei einer Temperatur zwischen 0 und +100°C und beinormalem Druck durchgeführt. Geeignete Lösungs- und Verdünnungsmittel sind u.a. Aether oder ätherartige Verbindungen wie z.B. Diäthyläther, Di-isopropyläther, Dioxan oder Tetrahydrofuran; aromatische Kohlenwasserstoffe wie Benzol, Toluol oder Xylole; Ketone wie Aceton, Methyläthylketon oder Cyclohexanon; oder chlorierte Kohlenwasserstoffe, wie Dichlormethan oder Tetrachlormethan.

Geeignete Basen können organischen oder anorganischen Ursprungs sein wie z.B. Natriumhydrid, Natrium- oder Calciumcarbonat, tertiäre Amine wie Triäthylamin, Triäthylendiamin oder 4-Dimethylaminopyridin oder Pyridin.

Eine andere Möglichkeit zur Herstellung der Isothiocyanate der Formel II besteht im Umweg über den entsprechenden, einseitig unsubstituierten Thioharnstoff. Dabei wird ein Anilin der Formel V mit Ammoniumrhodanid in saurem, vorzugsweise mineralsaurem Medium zum entsprechenden Thioharnstoff umgesetzt, der seinerseits beim Erhitzen auf +130°C bis 200°C Ammoniak abspaltet und in ein Isothiocyanat der Formel II übergeht (vgl. Saul Patai "The chemistry of cyanates and their thio derivatives", John Wiley and Sons, 1977; Chemistry and Industry, July 3, 1954, p. 735. J.N. Baxter et al., "New method of preparation of aryl isothiocyanates").

Die Aniline der Formel V ihrerseits können nach im Prinzip bekannten Methoden hergestellt werden, z.B. indem man ein Pyridin-2-thiol oder -thion der Formel VI

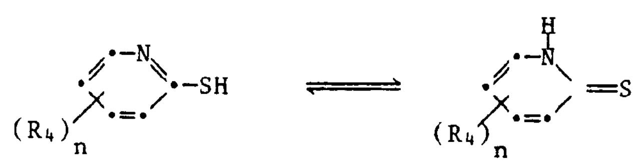 (VI)

mit einem 4-Halogenanilin der Formel VII

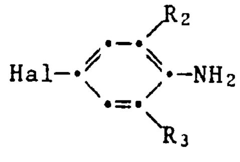 (VII)

umsetzt, wobei $R_2$, $R_3$, $R_4$ und n die Formel I angegebenen Bedeutungen haben und Hal für ein Halogenatom, insbesondere Chlor oder Brom, steht, und gegebenenfalls die Aminogruppe des entstandenen Pyridylthioanilins mit einer Schutzgruppe schützt, die Schwefelbrücke mit einem Oxydationsmittel in eine Sulfinyl- oder Sulfonylgruppe überführt und durch Hydrolyse die Aminogruppe wieder freisetzt.

Das Verfahren zur Herstellung der Pyridylthioaniline der Formel V wird zweckmässigerweise in Gegenwart einer anorganischen oder organischen Base wie z.B. einem Alkalihydroxid oder -carbonat, eines gegenüber den Reaktionsteilnehmern inerten, vorzugsweise polaren Lösungs- oder Verdünnungsmittels, und gegebenenfalls eines Katalysators wie z.B. Kupferpulver oder Kupfercarbonat bei einer Temperatur zwischen +80 und 160°C und unter normalem Druck durchgeführt. Geeignete Lösungs- und Verdünnungsmittel sind u.a. Aether oder ätherartige Verbindungen wie z.B. Diäthyläther, Di-isopropyläther, Dioxan oder Tetrahydrofuran; aromatische Kohlenwasserstoffe wie Benzol, Toluol oder Xylole; Ketone wie Aceton, Methyläthylketon oder Cyclohexanon; oder Alkohole wie Methanol oder Aethanol; besonders geeignet ist auch Dimethylsulfoxid oder Dimethylformamid. Als Oxydationsmittel für die Ueberführung der Schwefelbrücke in eine Sulfinyl- oder Sulfonylgruppe eignen sich u.a. $H_2O_2$/Eisessig, $H_2O_2$/Acetanhydrid, Persäuren wie z.B. m-Chlorbenzoepersäure, Perjodsäure oder Alkaliperjodate, Chromsäure oder Kaliumpermanganat. Als Schutz gruppen für die Aminogruppe des Phenylthioanilins eignen sich die üblichen, hydrolytisch oder hydrogenolytisch wieder abspaltbaren Schutzgruppen wie z.B. Acylgruppen (Acetyl, Trifluoracetyl), gegebenenfalls substituierte Benzylgruppen oder Alkoxyalkylgruppen (Methoxymethyl).

Die Verbindungen der Formeln II und V sind neu und bilden ebenfalls einen Gegenstand der vorliegenden Erfindung. Die Verbindungen der Formeln III, IV, VI und VII sind dagegen bekannt oder können nach im Prinzip bekannten Methoden hergestellt werden.

Ueberraschenderweise wurde gefunden, dass die erfindungsgemässen Verbindungen der Formel I bei günstiger Warmblüter- und Pflanzenverträglichkeit wertvolle Wirkstoffe in der Schädlingsbekämpfung sind. So eignen sich die Verbindungen der Formel I z.B. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und Arachniden der Ordnung Acarina, wie z.B. Milben und Zecken. Dabei kann jedes Entwicklungsstadium der Schädlinge bekämpft werden, d.h. sowohl die Adulten, Puppen und Nymphen, als auch insbesondere die Larven und Eier. So können vor allem Larven und Eier von phytopathogenen Schadinsekten und -milben in Zier- und Nutzpflanzungen, wie z.B. in Obst- und Gemüsepflanzungen, und insbesondere in Baumwollpflanzungen, wirkungsvoll bekämpft werden. Werden Verbindungen der Formel I von Imagines aufgenommen, so kann sich ihre Wirkung in unmittelbarer Abtötung der Schädlinge zeigen oder aber in verminderter Eiablage und/oder Schlupfrate. Die letztere Erscheinung kann insbesondere bei Coleopteren beobachtet werden. Bei der Bekämpfung von tierparasitären Schädlingen, insbesondere an Haus-und Nutztieren, kommen vor allem Ektoparasiten, wie z.B. Milben und Zecken und Dipteren, wie z.B. Lucilia sericata in Betracht.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit in der Formulierungstechnik üblichen, inerten und pflanzenverträglichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren

Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder adsorptive Granulatträger kommen poröse Typen, wie z.B. Bimsstein, Ziegelbruch Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit anderen Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979; Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung

1.1. Zwischenprodukte

1.1.1. Pyridylthioaniline

1.1.1.1. 2,6-Diäthyl-4-(3′,5′-dichlorpyrid-2′-ylthio)-anilin

15,0 g 2,6-Diäthyl-4-mercapto-anilin werden in einer Stickstoffatmosphäre bei Raumtemperatur tropfenweise zu 6,2 g pulverisiertem Kaliumhydroxid in 70 ml Dimethylsulfoxid gegeben. Unter ständigem Rühren wird nun eine Lösung von 15,8 g 2,3,5-Trichlorpyridin in 20 ml Dimethylsulfoxid tropfenweise zugegeben, wobei sich die Temperatur auf 45°C erhöht. Anschliessend wird das Reaktionsgemisch 3 Stunden lang nachgerührt, dann am Hochvakuum eingeengt und der Rückstand in je 80 ml Dichlormethan und Wasser aufgenommen. Die organische Phase wird mit Wasser gewaschen und über Natriumsulfat getrocknet. Dann wird das Lösungsmittel am Rotationsverdampfer entfernt und der Rückstand aus Toluol/Hexan umkristallisiert. Man erhält die Titelverbindung der Formel

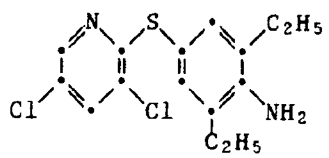

(Verb.1.1.1.1.)

als hell-beige Kristalle; Smp. 104-106°C.

Auf analoge Weise werden die folgenden Verbindungen hergestellt:

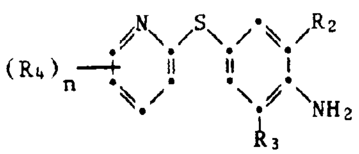

| Verb. Nr. | $R_2$ | $R_3$ | $R_4$ | n | phys. Daten |
|---|---|---|---|---|---|
| 1.1.1.2. | $CH(CH_3)_2$ | $CH(CH_3)_2$ | 3,5-$Cl_2$ | 2 | Smp. 79-81°C |
| 1.1.1.3. | $CH_3$ | $CH_3$ | 3,5-$Cl_2$ | 2 | Smp. 138-140°C |
| 1.1.1.4. | $C_2H_5$ | $CH(CH_3)C_2H_5$ | 3,5-$Cl_2$ | 2 | Smp. 90-92°C |
| 1.1.1.5. | $CH(CH_3)_2$ | $CH(CH_3)_2$ | 3-F,5-Cl | 2 | Smp. 83-84°C |
| | $C_2H_5$ | $C_2H_5$ | 5-Cl | 1 | |
| | $C_2H_5$ | $C_2H_5$ | 3-Cl,5-$CF_3$ | 2 | |
| | $C_2H_5$ | $C_2H_5$ | 3-F,5-Cl | 2 | |
| | $C_2H_5$ | $C_2H_5$ | 4,6-$Cl_2$ | 2 | |
| | $C_2H_5$ | $C_2H_5$ | 5-$CF_3$ | 1 | |
| | $C_2H_5$ | $CH_3$ | 3,5-$Cl_2$ | 2 | |
| | $C_2H_5$ | Cyclopentyl | 3,5-$Cl_2$ | 2 | |
| | $C_2H_5$ | Cyclohexyl | 3,5-$Cl_2$ | 2 | |
| | $CH(CH_3)_2$ | $CH(CH_3)_2$ | H | 1 | |
| | $CH(CH_3)_2$ | $CH(CH_3)_2$ | 3-Cl 5-$CF_2CFCl_2$ | 2 | |
| | $CH(CH_3)_2$ | $CH(CH_3)_2$ | 5-$CF_3$ | 1 | |
| | $CH(CH_3)_2$ | $CH(CH_3)_2$ | 3-Cl,5-$CH_3$ | 2 | |
| | $CH(CH_3)_2$ | $CH(CH_3)_2$ | 4,6-$Cl_2$ | 2 | |
| | $CH(CH_3)_2$ | $CH(CH_3)_2$ | 3-Cl,5-$CF_3$ | 2 | |
| | $CH(CH_3)_2$ | $CH(CH_3)_2$ | 5-Cl | 1 | |
| | $CH(CH_3)_2$ | Cyclopentyl | 3,5-$Cl_2$ | 2 | |
| | $CH(CH_3)_2$ | Cyclohexyl | 3,5-$Cl_2$ | 2 | |

1.1.2. Pyridylthio-phenylisothiocyanate

1.1.2.1. 2,6-Diäthyl-4-(3′,5′-dichlorpyrid-2′-ylthio)-phenylisothiocyanat

A) 6,3 g Thiophosgen, 9,2 g Calciumcarbonat und 60 ml Dichlormethan werden mit 40 ml Wasser verrührt. Zu diesem Gemisch wird bei 0 bis +10°C tropfenweise eine Lösung von 14,8 g 2,6-Diäthyl-4-(3′,5′-dichlorpyrid-2′-ylthio)-anilin in 30 ml Dichlormethan gegeben. Das Reaktionsgemisch wird 3 Stunden lang bei Raumtemperatur gerührt und danach filtriert. Aus dem Filtrat wird die organische Phase abgetrennt; diese wird zweimal mit je 30 ml Wasser gewaschen, über Natriumsulfat getrocknet und schliesslich eingedampft. Das Rohprodukt der Titelverbindung der Formel

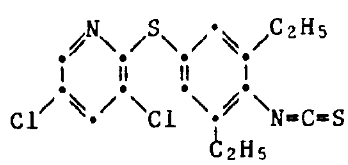

(Verb. Nr. 1.1.2.1.)

liegt als gelbbraunes Oel mit einem Brechungsindex von $n_D^{23}$: 1,6810 vor und kann so zur Weiterverarbeitung verwendet werden.

B) 9,9 g 2,6-Diäthyl-4-(3′,5′-dichlorpyrid-2-ylthio)-anilin, 2,3 g Ammoniumrhodanid, 4,5 ml konzentrierte Salzsäure und 70 ml o-Xylol werden gemischt und 3 Stunden lang unter Rühren bei +90°C gehalten. Anschliessend wird die Temperatur rasch auf +150°C erhöht und entweichendes Lösungsmittel, Wasser und Ammoniak aufgefangen. Nach 5 Stunden wird das Reaktionsgemisch auf Raumtemperatur abgekühlt, mit 40 ml Toluol versetzt und abfiltriert. Die Toluolphase wird mit 10 ml Wasser gewaschen, über Natriumsulfat getrocknet und eingedampft. Der Rückstand ist ein gelbbraunes Oel und entspricht der nach dem Verfahren A hergestellten Verbindung Nr. 1.1.2.1.

Auf analoge Weise werden die folgenden Verbindungen hergestellt:

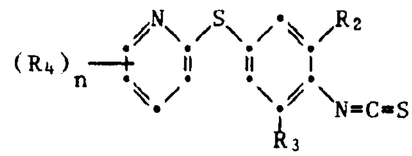

| Verb. Nr. | $R_2$ | $R_3$ | $R_4$ | n | phys. Daten |
|---|---|---|---|---|---|
| 1.1.2.2. | $CH(CH_3)_2$ | $CH(CH_3)_2$ | 3,5-$Cl_2$ | 2 | $n_D^{23}$: 1,6590 |
| 1.1.2.3. | $CH_3$ | $CH_3$ | 3,5-$Cl_2$ | 2 | Smp. 95-98°C |
| 1.1.2.4. | $C_2H_5$ | $CH(CH_3)C_2H_5$ | 3,5-$Cl_2$ | 2 | $n_D^{25}$: 1,6571 |
| 1.1.2.5. | $CH(CH_3)_2$ | $CH(CH_3)_2$ | 3-F,5-Cl | 2 | Oel |
| | $C_2H_5$ | $C_2H_5$ | 5-Cl | 1 | |
| | $C_2H_5$ | $C_2H_5$ | 3-Cl,5-$CF_3$ | 2 | |
| | $C_2H_5$ | $C_2H_5$ | 3-F,5-Cl | 2 | |
| | $C_2H_5$ | $C_2H_5$ | 4,6-$Cl_2$ | 2 | |
| | $C_2H_5$ | $C_2H_5$ | 5-$CF_3$ | 1 | |
| | $C_2H_5$ | $CH_3$ | 3,5-$Cl_2$ | 2 | |
| | $C_2H_5$ | Cyclopentyl | 3,5-$Cl_2$ | 2 | |
| | $C_2H_5$ | Cyclohexyl | 3,5-$Cl_2$ | 2 | |
| | $CH(CH_3)_2$ | $CH(CH_3)_2$ | H | 1 | |
| | $CH(CH_3)_2$ | $CH(CH_3)_2$ | 3-Cl<br>5-$CF_2CFCl_2$ | 2 | |
| | $CH(CH_3)_2$ | $CH(CH_3)_2$ | 5-$CF_3$ | 1 | |
| | $CH(CH_3)_2$ | $CH(CH_3)_2$ | 3-Cl,5-$CH_3$ | 2 | |
| | $CH(CH_3)_2$ | $CH(CH_3)_2$ | 4,6-$Cl_2$ | 2 | |
| | $CH(CH_3)_2$ | $CH(CH_3)_2$ | 3-Cl,5-$CF_3$ | 2 | |
| | $CH(CH_3)_2$ | $CH(CH_3)_2$ | 5-Cl | 1 | |
| | $CH(CH_3)_2$ | Cyclopentyl | 3,5-$Cl_2$ | 2 | |
| | $CH(CH_3)_2$ | Cyclohexyl | 3,5-$Cl_2$ | 2 | |

1.2 Endprodukte

1.2.1. Pyridylthio-phenylthioharnstoffe

1.2.1.1. N-[2,6-Diäthyl-4-(3′,5′-dichlorpyrid-2′-ylthio)-phenyl]-N′-tert.butyl-thioharnstoff

15,7 g 2,6-Diäthyl-4-(3′,5′-dichlorpyrid-2′-ylthio)-phenylisothiocyanat werden mit 120 ml Hexan verdünnt und bei +50°C tropfenweise mit 3,9 g tert.Butylamin versetzt. Das Reaktionsgemisch wird anschliessend bei +50°C 4 Stunden lang nachgerührt. Der entstandene Niederschlag wird abfiltriert und aus Toluol/Hexan umkristallisiert. Man erhält die Titelverbindung der Formel

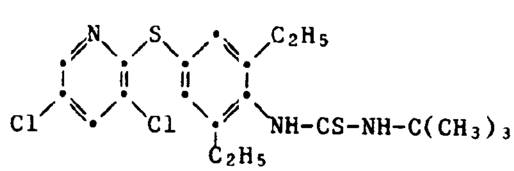

(Verb. Nr. 1.2.1.1.)

als hellbeiges Pulver; Smp. 140°C/Zers.

Auf analoge Weise werden die folgenden Verbindungen hergestellt:

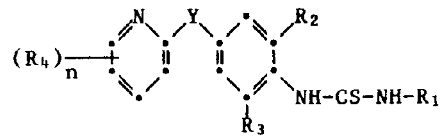

| Verb.Nr. | $R_1$ | $R_2$ | $R_3$ | $R_4$ | n | Y | Smp. °C |
|---|---|---|---|---|---|---|---|
| 1.2.1.2. | $C(CH_3)_3$ | $CH(CH_3)_2$ | $CH(CH_3)_2$ | 3,5-$Cl_2$ | 2 | S | 154/Zers. |
| 1.2.1.3. | $CH(CH_3)_2$ | $CH(CH_3)_2$ | CH(CH3)2 | 3,5-$Cl_2$ | 2 | S | 167-169 |
| 1.2.1.4. | Cyclopentyl | $CH(CH_3)_2$ | $CH(CH_3)_2$ | 3,5-$Cl_2$ | 2 | S | 172-174 |
| 1.2.1.5. | $CH(CH_3)C_2H_5$ | $CH(CH_3)_2$ | $CH(CH_3)_2$ | 3,5-$Cl_2$ | 2 | S | 134-136 |
| 1.2.1.6. | $C(CH_3)_2C_2H_5$ | $CH(CH_3)_2$ | $CH(CH_3)_2$ | 3,5-$Cl_2$ | 2 | S | 142-143 |
| 1.2.1.7. | $CH_3$ | $CH(CH_3)_2$ | $CH(CH_3)_2$ | 3,5-$Cl_2$ | 2 | S | 127-129 |
| 1.2.1.8. | $C(CH_3)_3$ | $CH_3$ | $CH_3$ | 3,5-$Cl_2$ | 2 | S | 147 u. Zers. |
| 1.2.1.9. | $C(CH_3)_3$ | $C_2H_5$ | $CH(CH_3)C_2H_5$ | 3,5-$Cl_2$ | 2 | S | 127-129 |
| 1.2.1.10. | $CH(CH_3)CH_2OCH_3$ | $CH(CH_3)_2$ | $CH(CH_3)_2$ | 3,5-$Cl_2$ | 2 | S | 125-126 |
| 1.2.1.11. | Cyclopropyl | $CH(CH_3)_2$ | $CH(CH_3)_2$ | 3,5-$Cl_2$ | 2 | S | 147-150 |
| 1.2.1.12. | Cyclohexyl | $CH(CH_3)_2$ | $CH(CH_3)_2$ | 3,5-$Cl_2$ | 2 | S | 174-177 |
| 1.2.1.13 | Cyclooctyl | $CH(CH_3)_2$ | $CH(CH_3)_2$ | 3,5-$Cl_2$ | 2 | S | 154-156 |
| 1.2.1.14 | $CH(CH_3)CH_2CH(CH_3)_2$ | $CH(CH_3)_2$ | $CH(CH_3)_2$ | 3,5-$Cl_2$ | 2 | S | 131-133 |
| 1.2.1.15 | $CH(CH_3)$Cyclopropyl | $CH(CH_3)_2$ | $CH(CH_3)_2$ | 3,5-$Cl_2$ | 2 | S | 138-140 |
| 1.2.1.16 | $C(CH_3)_3$ | $CH(CH_3)_2$ | $CH(CH_3)_2$ | 3-F,5-Cl | 2 | S | 115-116 |
| | $C_2H_5$ | $CH(CH_3)_2$ | $CH(CH_3)_2$ | 3,5-$Cl_2$ | 2 | S | |
| | $CH(CH_3)_2$ | $CH_3$ | $CH_3$ | 3,5-$Cl_2$ | 2 | S | |
| | $CH(CH_3)_2$ | $CH_3$ | $C_2H_5$ | 3,5-$Cl_2$ | 2 | S | |
| | $CH(CH_3)_2$ | $C_2H_5$ | $C_2H_5$ | 3,5-$Cl_2$ | 2 | S | |
| | $CH(CH_3)_2$ | $C_2H_5$ | $C_2H_5$ | 3,5-$Cl_2$ | 2 | SO | |
| | $CH(CH_3)_2$ | $C_2H_5$ | $C_2H_5$ | 3,5-$Cl_2$ | 2 | $SO_2$ | |
| | $CH(CH_3)_2$ | $C_2H_5$ | $C_2H_5$ | 5-Cl | 1 | S | |
| | $CH(CH_3)_2$ | $C_2H_5$ | $C_2H_5$ | 4,6-$Cl_2$ | 2 | S | |

| Verb.Nr. | $R_1$ | $R_2$ | $R_3$ | $R_4$ | n | Y |
|---|---|---|---|---|---|---|
| | $CH(CH_3)_2$ | $C_2H_5$ | $C_2H_5$ | 5-$CF_3$ | 1 | S |
| | $CH(CH_3)_2$ | $C_2H_5$ | $CH(CH_3)C_2H_5$ | 3,5-$Cl_2$ | 2 | S |
| | $CH(CH_3)_2$ | $CH(CH_3)_2$ | $CH(CH_3)_2$ | 3,5-$Cl_2$ | 2 | SO |
| | $CH(CH_3)_2$ | $CH(CH_3)_2$ | $CH(CH_3)_2$ | 3,5-$Cl_2$ | 2 | $SO_2$ |
| | $CH(CH_3)_2$ | $CH(CH_3)_2$ | $CH(CH_3)_2$ | 3-Cl,5-$CF_3$ | 2 | S |
| | $CH(CH_3)_2$ | $CH(CH_3)_2$ | $CH(CH_3)_2$ | 3-F,5-Cl | 2 | S |
| | $CH(CH_3)_2$ | $CH(CH_3)_2$ | $CH(CH_3)_2$ | 4,6-$Cl_2$ | 2 | S |
| | $C(CH_3)_3$ | $CH_3$ | $C_2H_5$ | 3,5-$Cl_2$ | 2 | S |
| | $C(CH_3)_3$ | $C_2H_5$ | $C_2H_5$ | 3,5-$Cl_2$ | 2 | SO |
| | $C(CH_3)_3$ | $C_2H_5$ | $C_2H_5$ | 3,5-$Cl_2$ | 2 | $SO_2$ |
| | $C(CH_3)_3$ | $C_2H_5$ | $C_2H_5$ | 5-Cl | 1 | S |
| | $C(CH_3)_3$ | $C_2H_5$ | $C_2H_5$ | 3-Cl,5-$CF_3$ | 2 | S |
| | $C(CH_3)_3$ | $C_2H_5$ | $C_2H_5$ | 3-F,5-Cl | 2 | S |
| | $C(CH_3)_3$ | $C_2H_5$ | $C_2H_5$ | 4,6-$Cl_2$ | 2 | S |
| | $C(CH_3)_3$ | $C_2H_5$ | Cyclopentyl | 3,5-$Cl_2$ | 2 | S |
| | $C(CH_3)_3$ | $C_2H_5$ | Cyclohexyl | 3,5-$Cl_2$ | 2 | S |
| | $C(CH_3)_3$ | $CH(CH_3)_2$ | $CH(CH_3)_2$ | 3,5-$Cl_2$ | 2 | SO |
| | $C(CH_3)_3$ | $CH(CH_3)_2$ | $CH(CH_3)_2$ | 3,5-$Cl_2$ | 2 | $SO_2$ |
| | $C(CH_3)_3$ | $CH(CH_3)_2$ | $CH(CH_3)_2$ | H | 1 | S |
| | $C(CH_3)_3$ | $CH(CH_3)_2$ | $CH(CH_3)_2$ | 5-$CF_3$ | 1 | S |
| | $C(CH_3)_3$ | $CH(CH_3)_2$ | $CH(CH_3)_2$ | 3-Cl,5-$CH_3$ | 2 | S |
| | $C(CH_3)_3$ | $CH(CH_3)_2$ | $CH(CH_3)_2$ | 4,6-$Cl_2$ | 2 | S |
| | $C(CH_3)_3$ | $CH(CH_3)_2$ | $CH(CH_3)_2$ | 3-Cl,5-$CF_3$ | 2 | S |
| | $C(CH_3)_3$ | $CH(CH_3)_2$ | $CH(CH_3)_2$ | 3-Cl, 5-$CF_2CFCl_2$ | 2 | S |
| | $C(CH_3)_3$ | $CH(CH_3)_2$ | $CH(CH_3)_2$ | 5-Cl | 1 | S |
| | $C(CH_3)_3$ | $CH(CH_3)_2$ | Cyclopentyl | 3,5-$Cl_2$ | 2 | S |
| | $C(CH_3)_3$ | $CH(CH_3)_2$ | Cyclohexyl | 3,5-$Cl_2$ | 2 | S |
| | $C(CH_3)_3$ | Cyclopent. | Cyclopentyl | 3,5-$Cl_2$ | 2 | S |
| | $CH(CH_3)C_2H_5$ | $C_2H_5$ | $C_2H_5$ | 3,5-$Cl_2$ | 2 | S |

| Verb.Nr. | $R_1$ | $R_2$ | $R_3$ | $R_4$ | n | Y |
|---|---|---|---|---|---|---|
| | $CH(CH_3)C_2H_5$ | $C_2H_5$ | $C_2H_5$ | 3,5-$Cl_2$ | 2 | SO |
| | $CH(CH_3)C_2H_5$ | $C_2H_5$ | $C_2H_5$ | 3,5-$Cl_2$ | 2 | $SO_2$ |
| | $CH(CH_3)C_2H_5$ | $CH(CH_3)_2$ | $CH(CH_3)_2$ | 3,5-$Cl_2$ | 2 | SO |
| | $CH(CH_3)C_2H_5$ | $CH(CH_3)_2$ | $CH(CH_3)_2$ | 3,5-$Cl_2$ | 2 | $SO_2$ |
| | $CH(C_2H_5)_2$ | $CH(CH_3)_2$ | $CH(CH_3)_2$ | 3,5-$Cl_2$ | 2 | S |
| | $CH[CH(CH_3)_2]_2$ | $CH(CH_3)_2$ | $CH(CH_3)_2$ | 3,5-$Cl_2$ | 2 | S |
| | $C_{12}H_{25}$ | $CH(CH_3)_2$ | $CH(CH_3)_2$ | 3,5-$Cl_2$ | 2 | S |
| | $CH_2CF_3$ | $CH(CH_3)_2$ | $CH(CH_3)_2$ | 3,5-$Cl_2$ | 2 | S |
| | $(CH_2)_3OC_4H_9$ | $CH(CH_3)_2$ | $CH(CH_3)_2$ | 3,5-$Cl_2$ | 2 | S |
| | $CH(CH_3)$Cyclo-hexyl | $CH(CH_3)_2$ | $CH(CH_3)_2$ | 3,5-$Cl_2$ | 2 | S |
| | $C(CH_3)_2$Cyclo-hexyl | $CH(CH_3)_2$ | $CH(CH_3)_2$ | 3,5-$Cl_2$ | 2 | S |
| | Cyclopentyl | $C_2H_5$ | $C_2H_5$ | 3,5-$Cl_2$ | 2 | S |
| | Cyclopentyl | $C_2H_5$ | $C_2H_5$ | 3,5-$Cl_2$ | 2 | SO |
| | Cyclopentyl | $C_2H_5$ | $C_2H_5$ | 3,5-$Cl_2$ | 2 | $SO_2$ |
| | Cyclopentyl | $CH(CH_3)_2$ | $CH(CH_3)_2$ | 3,5-$Cl_2$ | 2 | S |
| | Cyclopentyl | $CH(CH_3)_2$ | $CH(CH_3)_2$ | 3,5-$Cl_2$ | 2 | $SO_2$ |
| | 2,6-Diäthyl-Cyclohexyl | $CH(CH_3)_2$ | $CH(CH_3)_2$ | 3,5-$Cl_2$ | 2 | S |

1.2.2. Pyridylthio-phenylisothioharnstoffe

1.2.2.1. N-[2,6-Diäthyl-4-(3′,5′-dichlorpyrid-2′-ylthio)-phenyl]-N′-tert.butyl-S-methyl-isothioharnstoff

5,0 g N-[2,6-Diäthyl-4-(3′,5′-dichlorpyrid-2′-ylthio)-phenyl]N′-tert.butyl-thioharnstoff werden in 50 ml Aethanol vorgelegt, bei Raumtemperatur mit 2,2 g Methyljodid versetzt und während 3 Stunden unter leichtem Rückfluss gerührt. Anschliessend wird die Reaktionslösung eingedampft und der Rückstand in 100 ml Dichlormethan und 50 ml 10%iger Natriumcarbonatlösung aufgenommen. Die organische Phase wird abgetrennt, mit Wasser gewaschen, über Natriumsulfat getrocknet und zur Trockne eingedampft. Der Rückstand wird aus Hexan umkristallisiert. Man erhält die Titelverbindung der Formel

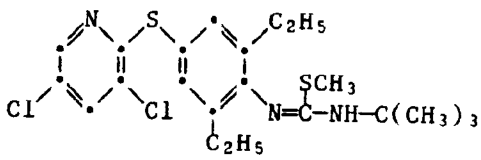

(Verb.Nr.1.2.2.1.)

in Form von gelblich-beigen Kristallen; Smp. 122-124°C.

Auf analoge Weise werden die folgenden Verbindungen hergestellt:

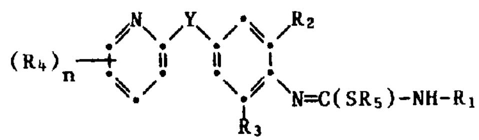

| Verb.Nr. | $R_1$ | $R_2$ | $R_3$ | $R_4$ | $R_5$ | n | Y | phys. Daten |
|---|---|---|---|---|---|---|---|---|
| 1.2.2.2. | $C(CH_3)_3$ | $CH(CH_3)_2$ | $CH(CH_3)_2$ | 3,5-$Cl_2$ | $CH_3$ | 2 | S | Smp. 105-107°C |
| 1.2.2.3. | $CH(CH_3)C_2H_5$ | $CH(CH_3)_2$ | $CH(CH_3)_2$ | 3,5-$Cl_2$ | $CH_3$ | 2 | S | Smp. 100-103°C |
| 1.2.2.4. | $C(CH_3)_2C_2H_5$ | $CH(CH_3)_2$ | $CH(CH_3)_2$ | 3,5-$Cl_2$ | $CH_3$ | 2 | S | Smp. 106-108°C |
| 1.2.2.5. | Cyclopentyl | $CH(CH_3)_2$ | $CH(CH_3)_2$ | 3,5-$Cl_2$ | $CH_3$ | 2 | S | Smp. 80- 83°C |
| 1.2.2.6. | $C(CH_3)_3$ | $C_2H_5$ | $CH(CH_3)C_2H_5$ | 3,5-$Cl_2$ | $CH_3$ | 2 | S | $n_D^{40}$: 1,5890 |
| 1.2.2.7. | $CH(CH_3)_2$ | $C_2H_5$ | $CH(CH_3)C_2H_5$ | 3,5-$Cl_2$ | $CH_3$ | 2 | S | $n_D^{40}$: 1,6014 |
| | $CH(CH_3)_2$ | $C_2H_5$ | $C_2H_5$ | 3,5-$Cl_2$ | $C_2H_5$ | 2 | S | |
| | $CH(CH_3)_2$ | $C_2H_5$ | $C_2H_5$ | 3,5-$Cl_2$ | $C_3H_7$ | 2 | S | |
| | $CH(CH_3)_2$ | $C_2H_5$ | $C_2H_5$ | 3,5-$Cl_2$ | $C_4H_9$ | 2 | S | |
| | $CH(CH_3)_2$ | $CH(CH_3)_2$ | $CH(CH_3)_2$ | 3,5-$Cl_2$ | $C_2H_5$ | 2 | S | |
| | $CH(CH_3)_2$ | $CH(CH_3)_2$ | $CH(CH_3)_2$ | 3,5-$Cl_2$ | $CH(CH_3)_2$ | 2 | S | |
| | $C(CH_3)_3$ | $C_2H_5$ | $C_2H_5$ | 3,5-$Cl_2$ | $C_2H_5$ | 2 | S | |
| | $C(CH_3)_3$ | $CH(CH_3)_2$ | $CH(CH_3)_2$ | 3,5-$Cl_2$ | $C_2H_5$ | 2 | S | |
| | $C(CH_3)_3$ | $CH(CH_3)_2$ | $CH(CH_3)_2$ | 3,5-$Cl_2$ | $C_2H_5$ | 2 | SO | |
| | $C(CH_3)_3$ | $CH(CH_3)_2$ | $CH(CH_3)_2$ | 3,5-$Cl_2$ | $C_2H_5$ | 2 | $SO_2$ | |
| | $C(CH_3)_3$ | $CH(CH_3)_2$ | $CH(CH_3)_2$ | 3,5-$Cl_2$ | $C_3H_7$ | 2 | S | |
| | $C(CH_3)_3$ | $CH(CH_3)_2$ | $CH(CH_3)_2$ | 3,5-$Cl_2$ | $C_4H_9$ | 2 | S | |
| | $C(CH_3)_3$ | $CH(CH_3)_2$ | $CH(CH_3)_2$ | 3,5-$Cl_2$ | $C_6H_{13}$ | 2 | S | |
| | $C(CH_3)_3$ | $CH(CH_3)_2$ | $CH(CH_3)_2$ | 3,5-$Cl_2$ | $C_8H_{17}$ | 2 | S | |
| | $C(CH_3)_3$ | $CH(CH_3)_2$ | $CH(CH_3)_2$ | 3,5-$Cl_2$ | $C_{10}H_{21}$ | 2 | S | |
| | $C(CH_3)_3$ | $CH(CH_3)_2$ | $CH(CH_3)_2$ | 3,5-$Cl_2$ | Allyl | 2 | S | |

1.2.3. Pyridylthio-phenylcarbodiimide

1.2.3.1. N-[2,6-Diäthyl-4-(3',5'-dichlorpyrid-2'-ylthio)-phenyl]-N'-tert.butyl-carbodiimid

5,0 g N-[2,6-Diäthyl-4-(3',5'-dichlorpyrid-2'-ylthio)-phenyl]-N'-tert.butyl 1-thioharnstoff und 3,5 g 2-Chlor-1-methylpyridiniumjodid werden in 25 ml Acetonitril vorgelegt, bei Raumtemperatur tropfenweise unter Rühren mit 2,7 g Triäthylamin in 10 ml Acetonitril versetzt und während 2 Stunden bei +70°C Badtemperatur gerührt. Anschliessend wird das Reaktionsgemisch am Rotationsverdampfer bei +50°C eingedampft und der Rückstand in 50 ml Hexan und 30 ml Wasser aufgenommen. Die Hexanphase wird abgetrennt, mit Wasser gewaschen, über Natriumsulfat getrocknet und schliesslich eingedampft. Man erhält die Titelverbindung der Formel

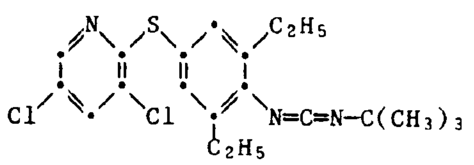

(Verb.Nr.1.2.3.1.)

als gelbliches Oel; Brechungsindex $n_D^{25}$: 1,6129.

Auf analoge Weise werden die folgenden Verbindungen hergestellt:

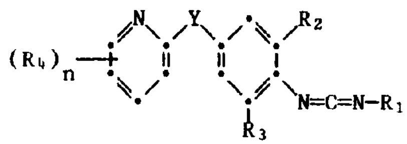

| Verb.Nr. | $R_1$ | $R_2$ | $R_3$ | $R_4$ | n | Y | phys. Daten |
|---|---|---|---|---|---|---|---|
| 1.2.3.2. | $C(CH_3)_3$ | $CH(CH_3)_2$ | $CH(CH_3)_2$ | 3,5-$Cl_2$ | 2 | S | Smp.58-60°C |
| 1.2.3.3. | $CH(CH_3)_2$ | $CH(CH_3)_2$ | CH(CH3)$_2$ | 3,5-$Cl_2$ | 2 | S | $n_D^{25}$: 1,6070 |
| 1.2.3.4. | $CH(CH_3)C_2H_5$ | $CH(CH_3)_2$ | $CH(CH_3)_2$ | 3,5-$Cl_2$ | 2 | S | $n_D^{25}$: 1,6011 |
| 1.2.3.5. | $C(CH_3)_2C_2H_5$ | $CH(CH_3)_2$ | $CH(CH_3)_2$ | 3,5-$Cl_2$ | 2 | S | $n_D^{25}$: 1,5959 |
| 1.2.3.6. | Cyclopentyl | $CH(CH_3)_2$ | $CH(CH_3)_2$ | 3,5-$Cl_2$ | 2 | S | $n_D^{25}$: 1,6081 |
| 1.2.3.7. | $CH_3$ | $CH(CH_3)_2$ | $CH(CH_3)_2$ | 3,5-$Cl_2$ | 2 | S | $n_D^{25}$: 1,624 |
| 1.2.3.8. | $CH(CH_3)_2$ | $C_2H_5$ | $CH(CH_3)C_2H_5$ | 3,5-$Cl_2$ | 2 | S | $n_D^{25}$: 1,6045 |
| 1.2.3.9. | $C(CH_3)_3$ | $CH_3$ | $CH_3$ | 3,5-$Cl_2$ | 2 | S | $n_D^{25}$: 1,6256 |
| 1.2.3.10. | $C(CH_3)_3$ | $C_2H_5$ | $CH(CH_3)C_2H_5$ | 3,5-$Cl_2$ | 2 | S | $n_D^{25}$: 1,5961 |
| 1.2.3.11. | $C(CH_3)_3$ | $CH(CH_3)_2$ | $CH(CH_3)_2$ | 3-F,5-Cl | 2 | S | $n_D^{25}$: 1,5804 |
| 1.2.3.12. | $CH(CH_3)CH_2OCH_3$ | $CH(CH_3)_2$ | $CH(CH_3)_2$ | 3,5-$Cl_2$ | 2 | S | $n_D^{24}$: 1,6006 |
| 1.2.3.13. | Cyclopropyl | $CH(CH_3)_2$ | $CH(CH_3)_2$ | 3,5-$Cl_2$ | 2 | S | $n_D^{24}$: 1,622 |

| Verb.Nr. | $R_1$ | $R_2$ | $R_3$ | $R_4$ | n | Y | phys. Daten |
|---|---|---|---|---|---|---|---|
| 1.2.3.14. | 2,6-Diäthyl-cyclohexyl | $CH(CH_3)_2$ | $CH(CH_3)_2$ | 3,5-$Cl_2$ | 2 | S | $n_D^{24}$: 1,610 |
| 1.2.3.15. | Cyclooctyl | $CH(CH_3)_2$ | $CH(CH_3)_2$ | 3,5-$Cl_2$ | 2 | S | $n_D^{24}$: 1,607 |
| 1.2.3.16. | $CH(CH_3)CH_2CH(CH_3)_2$ | $CH(CH_3)_2$ | $CH(CH_3)_2$ | 3,5-$Cl_2$ | 2 | S | $n_D^{25}$: 1,5891 |
| 1.2.3.17. | $CH(CH_3)$Cyclopropyl | $CH(CH_3)_2$ | $CH(CH_3)_2$ | 3,5-$Cl_2$ | 2 | S | $n_D^{25}$: 1,6072 |
| | $C_2H_5$ | $CH(CH_3)_2$ | $CH(CH_3)_2$ | 3,5-$Cl_2$ | 2 | S | |
| | $CH(CH_3)_2$ | $CH_3$ | $CH_3$ | 3,5-$Cl_2$ | 2 | S | |
| | $CH(CH_3)_2$ | $CH_3$ | $C_2H_5$ | 3,5-$Cl_2$ | 2 | S | |
| | $CH(CH_3)_2$ | $C_2H_5$ | $C_2H_5$ | 3,5-$Cl_2$ | 2 | S | |
| | $CH(CH_3)_2$ | $C_2H_5$ | $C_2H_5$ | 3,5-$Cl_2$ | 2 | SO | |
| | $CH(CH_3)_2$ | $C_2H_5$ | $C_2H_5$ | 3,5-$Cl_2$ | 2 | $SO_2$ | |
| | $CH(CH_3)_2$ | $C_2H_5$ | $C_2H_5$ | 5-Cl | 1 | S | |
| | $CH(CH_3)_2$ | $C_2H_5$ | $C_2H_5$ | 4,6-$Cl_2$ | 2 | S | |
| | $CH(CH_3)_2$ | $C_2H_5$ | $C_2H_5$ | 5-$CF_3$ | 1 | S | |
| | $CH(CH_3)_2$ | $CH(CH_3)_2$ | $CH(CH_3)_2$ | 3,5-$Cl_2$ | 2 | SO | |
| | $CH(CH_3)_2$ | $CH(CH_3)_2$ | $CH(CH_3)_2$ | 3,5-$Cl_2$ | 2 | $SO_2$ | |
| | $CH(CH_3)_2$ | $CH(CH_3)_2$ | $CH(CH_3)_2$ | 3-Cl,5-$CF_3$ | 2 | S | |
| | $CH(CH_3)_2$ | $CH(CH_3)_2$ | $CH(CH_3)_2$ | 3-F,5-Cl | 2 | S | |
| | $CH(CH_3)_2$ | $CH(CH_3)_2$ | $CH(CH_3)_2$ | 4,6-$Cl_2$ | 2 | S | |
| | $C(CH_3)_3$ | $CH_3$ | $C_2H_5$ | 3,5-$Cl_2$ | 2 | S | |
| | $C(CH_3)_3$ | $C_2H_5$ | $C_2H_5$ | 3,5-$Cl_2$ | 2 | SO | |
| | $C(CH_3)_3$ | $C_2H_5$ | $C_2H_5$ | 3,5-$Cl_2$ | 2 | $SO_2$ | |
| | $C(CH_3)_3$ | $C_2H_5$ | $C_2H_5$ | 5-Cl | 1 | S | |
| | $C(CH_3)_3$ | $C_2H_5$ | $C_2H_5$ | 3-Cl,5-$CF_3$ | 2 | S | |
| | $C(CH_3)_3$ | $C_2H_5$ | $C_2H_5$ | 3-F,5-Cl | 2 | S | |
| | $C(CH_3)_3$ | $C_2H_5$ | $C_2H_5$ | 4,6-$Cl_2$ | 2 | S | |

| Verb.Nr. | $R_1$ | $R_2$ | $R_3$ | $R_4$ | n | Y |
|---|---|---|---|---|---|---|
| | $C(CH_3)_3$ | $C_2H_5$ | Cyclopentyl | 3,5-$Cl_2$ | 2 | S |
| | $C(CH_3)_3$ | $C_2H_5$ | Cyclohexyl | 3,5-$Cl_2$ | 2 | S |
| | $C(CH_3)_3$ | $CH(CH_3)_2$ | $CH(CH_3)_2$ | 3,5-$Cl_2$ | 2 | SO |
| | $C(CH_3)_3$ | $CH(CH_3)_2$ | $CH(CH_3)_2$ | 3,5-$Cl_2$ | 2 | $SO_2$ |
| | $C(CH_3)_3$ | $CH(CH_3)_2$ | $CH(CH_3)_2$ | H | 1 | S |
| | $C(CH_3)_3$ | $CH(CH_3)_2$ | $CH(CH_3)_2$ | 5-$CF_3$ | 1 | S |
| | $C(CH_3)_3$ | $CH(CH_3)_2$ | $CH(CH_3)_2$ | 3-Cl,5-$CH_3$ | 2 | S |
| | $C(CH_3)_3$ | $CH(CH_3)_2$ | $CH(CH_3)_2$ | 4,6-$Cl_2$ | 2 | S |
| | $C(CH_3)_3$ | $CH(CH_3)_2$ | $CH(CH_3)_2$ | 3-Cl,5-$CF_3$ | 2 | S |
| | $C(CH_3)_3$ | $CH(CH_3)_2$ | $CH(CH_3)_2$ | 3-Cl, 5-$CF_2CFCl_2$ | 2 | S |
| | $C(CH_3)_3$ | $CH(CH_3)_2$ | $CH(CH_3)_2$ | 5-Cl | 1 | S |
| | $C(CH_3)_3$ | $CH(CH_3)_2$ | Cyclopentyl | 3,5-$Cl_2$ | 2 | S |
| | $C(CH_3)_3$ | $CH(CH_3)_2$ | Cyclohexyl | 3,5-$Cl_2$ | 2 | S |
| | $C(CH_3)_3$ | Cyclopent. | Cyclopentyl | 3,5-$Cl_2$ | 2 | S |
| | $CH(CH_3)C_2H_5$ | $C_2H_5$ | $C_2H_5$ | 3,5-$Cl_2$ | 2 | S |
| | $CH(CH_3)C_2H_5$ | $C_2H_5$ | $C_2H_5$ | 3,5-$Cl_2$ | 2 | SO |
| | $CH(CH_3)C_2H_5$ | $C_2H_5$ | $C_2H_5$ | 3,5-$Cl_2$ | 2 | $SO_2$ |
| | $CH(CH_3)C_2H_5$ | $CH(CH_3)_2$ | $CH(CH_3)_2$ | 3,5-$Cl_2$ | 2 | SO |
| | $CH(CH_3)C_2H_5$ | $CH(CH_3)_2$ | $CH(CH_3)_2$ | 3,5-$Cl_2$ | 2 | $SO_2$ |
| | $CH(C_2H_5)_2$ | $CH(CH_3)_2$ | $CH(CH_3)_2$ | 3,5-$Cl_2$ | 2 | S |
| | $CH[CH(CH_3)_2]_2$ | $CH(CH_3)_2$ | $CH(CH_3)_2$ | 3,5-$Cl_2$ | 2 | S |
| | $C_{12}H_{25}$ | $CH(CH_3)_2$ | $CH(CH_3)_2$ | 3,5-$Cl_2$ | 2 | S |
| | $CH_2CF_3$ | $CH(CH_3)_2$ | $CH(CH_3)_2$ | 3,5-$Cl_2$ | 2 | S |
| | $(CH_2)_3OC_4H_9$ | $CH(CH_3)_2$ | $CH(CH_3)_2$ | 3,5-$Cl_2$ | 2 | S |
| | $CH(CH_3)$Cyclohexyl | $CH(CH_3)_2$ | $CH(CH_3)_2$ | 3,5-$Cl_2$ | 2 | S |
| | $C(CH_3)_2$Cyclohexyl | $CH(CH_3)_2$ | $CH(CH_3)_2$ | 3,5-$Cl_2$ | 2 | S |
| | Cyclopentyl | $C_2H_5$ | $C_2H_5$ | 3,5-$Cl_2$ | 2 | S |
| | Cyclopentyl | $C_2H_5$ | $C_2H_5$ | 3,5-$Cl_2$ | 2 | SO |

| Verb.Nr. | $R_1$ | $R_2$ | $R_3$ | $R_4$ | n | Y |
|---|---|---|---|---|---|---|
| | Cyclopentyl | $C_2H_5$ | $C_2H_5$ | 3,5-$Cl_2$ | 2 | $SO_2$ |
| | Cyclopentyl | $CH(CH_3)_2$ | $CH(CH_3)_2$ | 3,5-$Cl_2$ | 2 | S |
| | Cyclopentyl | $CH(CH_3)_2$ | $CH(CH_3)_2$ | 3,5-$Cl_2$ | 2 | $SO_2$ |
| | Cyclohexyl | $CH(CH_3)_2$ | $CH(CH_3)_2$ | 3,5-$Cl_2$ | 2 | S |

Beispiel 2: Formulierungen von Wirkstoffen der Formel I gemäss den Herstellungsbeispielen 1.2. (% = Gewichtsprozent)

| 2.1. Emulsions-Konzentrate | a) | b) |
|---|---|---|
| Wirkstoff gemäss den Herstellungsbeispielen 1.2. | 10 % | 25 % |
| Ca-Dodecylbenzolsulfonat | - | 5 % |
| Ricinusöl-polyäthylenglykoläther (36 Mol AeO) | 25 % | 5 % |
| Cyclohexanon | - | 40 % |
| Butanol | 15 % | - |
| Xylolgemisch | - | 25 % |
| Essigester | 50 % | - |

Aus solchen Konzentraten können durch Verdünnen mit Wasser Emulsionen jeder gewünschten Konzentration hergestellt werden.

| 2.2. Lösungen | a) | b) |
|---|---|---|
| Wirkstoff gemäss den Herstellungsbeispielen 1.2. | 10 % | 5 % |
| Polyäthylenglykol (MG 400) | 70 % | - |
| N-Methyl-2-pyrrolidon | 20 % | 20 % |
| Epoxidiertes Kokosnussöl | - | 1 % |
| Benzin (Siedegrenzen 160-190°C) | - | 74 % |

Die Lösungen sind zur Anwendung in Form kleinster Tropfen geeignet.

| 2.3. Granulate | a) | b) |
|---|---|---|
| Wirkstoff gemäss den Herstellungsbeispielen 1.2. | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

2.4. Extruder Granulat

| | |
|---|---|
| Wirkstoff gemäss den Herstellungsbeispielen 1.2. | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.5. Umhüllungs-Granulat

| | |
|---|---|
| Wirkstoff gemäss den Herstellungsbeispielen 1.2. | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 2.6. Stäubemittel | a) | b) | c) | d) |
|---|---|---|---|---|
| Wirkstoff gemäss den Herstellungsbeispielen 1.2. | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoffe und gegebenenfalls Vermahlen auf einer geeigneten Mühle erhält man gebrauchsfertige Stäubemittel.

| 2.7. Spritzpulver | a) | b) | c) |
|---|---|---|---|
| Wirkstoff gemäss den Herstellungsbeispielen 1.2. | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoffe wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

| 2.8. Suspensions-Konzentrat | |
|---|---|
| Wirkstoff gemäss den Herstellungsbeispielen 1.2. | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Biologische Prüfungen

3.1. Wirkung gegen Musca domestica

Ein Zuckerwürfel wird derart mit einer Lösung der Testsubstanz befeuchtet, dass die Konzentration des Wirkstoffes im Würfel nach dem Trocknen 500 ppm beträgt. Der so behandelte Würfel wird zusammen mit einem nassen Wattebausch auf eine Schale gelegt und mit einem Becherglas zugedeckt. Unter das Becherglas werden 10 adulte, eine Woche alte und OP-resistente Fliegen gegeben und bei 25°C und 50 % Luftfeuchtigkeit belassen. Nach 24 Stunden wird die insektizide Wirkung durch Ermittlung der Abtötungsrate festgestellt.

Verbindungen gemäss den Beispielen 1.2.1., 1.2.2. und 1.2.3. zeigen gute Wirkung im obigen Test.

3.2. Wirkung gegen Lucilia sericata

Zu 9 ml eines Zuchtmediums wird bei 50°C 1 ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen gemäss den Beispielen 1.2.1., 1,2,2. und 1.2.3. zeigen in diesem Test gute Wirkung gegen Lucilia sericata.

3.3. Wirkung gegen Zecken in verschiedenen Entwicklungsstadien

10 frische, vollgesogene Boophilus microplus Weibchen werden in einer Reihe dorsal auf einer PVC Platte aufgeklebt und mit einem Wattebausch überdeckt. Danach werden die Testtiere mit 10 ml der wässrigen Testlösung übergossen. Eine Stunde später wird der Wattebausch entfernt und die Zecken über Nacht bei 24°C getrocknet. Nach dem Trocknen werden die Zecken 4 Woche lang bis zum Ende der Eiablage und Beginn des Larvenschlupfes bei 28°C und 80 % Luftfeuchtigkeit gehalten.

Jede Testsubstanz wird in einer Konzentration von 500 ppm getestet. Die akarizide Wirkung zeigt sich entweder beim Weibchen als Mortalität oder Sterilität oder im Eigelege durch Blockierung der Embryogenese oder des Schlüpfaktes. Alle Substanzen werden gegen zwei verschiedene Zeckenstämme, den OP-resistenten Stamm BIARRA und den Amidin-resistenten Stamm ULAM geprüft.

Verbindungen gemäss den Beispielen 1.2.1., 1.2.2. und 1.2.3. zeigen gute Wirkung im obigen Test.

3.4. Frassgift-Wirkung auf Spodoptera littoralis-Larven ($L_1$)

Baumwollpflanzen im Keimblattstadium werden mit einer wässrigen Wirkstoffemulsion (erhalten aus einem 10%igen emulgierbaren Konzentrat) besprüht, wobei die Wirkstoffemulsion 400 ppm der zu prüfenden Verbindung enthält.

Nach dem Antrocknen des Belages wird jede Baumwollpflanze mit Spodoptera littoralis-Larven im ersten larvalen Stadium besetzt. Der Versuch wird bei 26°C und ca. 50% relativer Luftfeuchtigkeit durchgeführt. Nach 2 und 3 Tagen wird die Mortalität und nach 5 Tagen werden Entwicklungs- und Häutungsstörungen der angesetzten Larven bestimmt.

Verbindungen gemäss den Beispielen 1.2.1., 1.2.2. und 1.2.3. zeigen gute Wirkung in diesem Test.

3.5. Frassgift-Wirkung auf Spodoptera littoralis- und Heliothis virescens-Larven ($L_3$)

Eingetropfte Sojapflanzen (Topfgrösse 10 cm Durchmesser) im 4-Blatt-Stadium werden mit wässrigen Wirkstoffemulsionen besprüht, die den Wirkstoff in einer Konzentration von 400 ppm enthalten.

Nach zwei Tagen werden die behandelten Sojapflanzen mit je 10 Larven von Spodoptera littoralis oder Heliothis virescens im dritten larvalen Stadium besiedelt. Der Versuch wird bei 26°C und ca. 60 % relativer Luftfeuchtigkeit im Dämmerlicht durchgeführt. Die Bonitur erfolgt nach 2 und 5 Tagen; es wird die Mortalität der Larven in Prozenten bestimmt.

Verbindungen gemäss den Beispielen 1.2.1., 1,2,2. und 1.2.3. zeigen 80-100%ige Wirkung (Mortalität).

3.6. Frassgift-Wirkung auf Plutella xylostella-Larven ($L_2$)

Eingetropfte Chinakohlpflanzen (Topfgrösse 10 cm Durchmesser) im 4-Blatt-Stadium werden mit wässrigen Wirkstoffemulsionen besprüht, die den Wirkstoff in einer Konzentration von 400 ppm enthalten.

Nach zwei Tagen werden die behandelten Chinakohlpflanzen mit 10 Plutella xylostella-Larven im zweiten larvalen Stadium besiedelt. Der Versuch wird bei 26°C und ca. 60 % relativer Luftfeuchtigkeit im Dämmerlicht durchgeführt. Die Bonitur erfolgt nach 2 und 5 Tagen; es wird die Mortalität der Larven in Prozenten bestimmt.

Verbindungen gemäss den Beispielen 1.2.1., 1.2.2. und 1.2.3. zeigen 80-100%ige Wirkung (Mortalität).

3.7. Kontaktwirkung gegen Nilaparvata lugens (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 40 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu

hindern, wird über die besiedelten Pflanzen jeweils ein Plexiglaszylinder gestülpt und dieser mit einem Gaze-Deckel abgedeckt. Die Nymphen werden für 6 Tage an der behandelten Pflanze, die mindestens 1 mal nachgegossen werden muss, gehalten. Der Versuch wird bei einer Temperatur von ca. 23°C, bei 55 % relativer Luftfeuchtigkeit und mit einer Beleuchtungsperiode von 16 Stunden durchgeführt.

Verbindungen gemäss den Beispielen 1.2.1., 1.2.2. und 1.2.3. zeigen in diesem Test gute Wirkung.

3.8. Systemische Wirkung auf Nilaparvata lugens

Etwa 10 Tage alte Reispflanzen (ca. 10 cm hoch) werden in einen Plastik-Becher eingestellt, der 20 ml einer wässrigen Emulsions-Zubereitung des zu prüfenden Wirkstoffes in einer Konzentration von 100 ppm enthält und mit einem Löcher aufweisenden Plastikdeckel abgeschlossen ist. Die Wurzel der Reispflanze wird durch ein Loch in dem Plastikdeckel in die wässrige Test-Zubereitung geschoben. Das Loch wird mit Watte abgedichtet, um die Pflanze zu fixieren und den Einfluss der Gasphase aus der Test-Zubereitung auszuschalten. Dann wird die Reispflanze mit 20 Nymphen von Nilaparvata lugens im $N_2$-bis $N_3$- Stadium besiedelt und mit einem Plastikzylinder abgedeckt. Der Versuch wird bei 26°C und ca. 60 % relativer Luftfeuchtigkeit mit einer Beleuchtungsperiode von 16 Stunden durchgeführt. Nach zwei und fünf Tagen wird auf die Anzahl der abgetöteten Testtiere, im Vergleich zu unbehandelten Kontrollen, bonitiert. Damit wird festgestellt, ob der über die Wurzel aufgenommene Wirkstoff, die Testtiere an den oberen Pflanzenteilen abtötet.

Verbindungen gemäss den Beispielen 1.2.1., 1.2.2. und 1.2.3. zeigen im obigen Test 80 - 100%ige Wirkung (Mortalität) gegen Nilaparvata lugens.

3.9 Wirkung gegen Bodeninsekten (Diabrotica balteata)

Es werden 350 ml Erde (bestehend aus 95 Vol.-% Sand und 5 Vol.-% Torf) mit jeweils 150 ml von wässrigen Emulsionszubereitungen vermischt, die den zu prüfenden Wirkstoff in einer Konzentration von 400 ppm enthalten. Dann werden Plastikbecher, die einen oberen Durchmesser von ca. 10 cm haben, mit der so behandelten Erde teilweise gefüllt. Pro Becher werden zehn Larven von Diabrotica balteata im dritten Larvalstadium eingesetzt, vier Maiskeimlinge eingepflanzt und die Becher mit Erde aufgefüllt. Die gefüllten Becher werden mit Plastikfolie abgedeckt und bei einer Temperatur von etwa 24°C und einer relativen Luftfeuchtigkeit von ca. 50 % gehalten. Sechs Tage nach dem Ansatz wird die in den Bechern enthaltenden Erde abgesiebt und die zurückbleibenden Larven werden auf ihre Mortalität geprüft.

Verbindungen gemäss den Beispielen 1.2.1., 1.2.2. und 1.2.3. zeigen gute Wirkung im obigen Test.

3.10. Kontaktwirkung auf Aphis craccivora

In Töpfen angezogene 4-5 Tage alte Erbsenkeimlinge (Pisum sativum) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 400 ppm der zu prüfenden Verbindung bis zur Tropfnässe direkt besprüht. Man verwendet pro Test-Verbindung zwei Pflanzen. Eine Auswertung der erzielten Abtötungsrate erfolgt nach 3 und 5 Tagen. Der Versuch wird bei ca. 21°C und einer relativen Luftfeuchtigkeit von etwa 55 % durchgeführt.

Verbindungen gemäss den Beispielen 1.2.1., 1.2.2. und 1.2.3. zeigen gute Wirkung in diesem Test.

3.11. Kontaktwirkung auf Myzus persicae

Etwa 4 bis 5 Tage alte, in Wasser angezogene Erbsenkeimlinge (Pisum sativum) werden vor Versuchsbeginn jeweils mit ca. 200 Individuen der Spezies Myzus persicae besiedelt. Die so behandelten Pflanzen werden 24 Stunden später mit einer wässrigen Suspension enthaltend 100 ppm der zu prüfenden Verbindung bis zur Tropfnässe direkt besprüht. Man verwendet pro Testsubstanz zwei Pflanzen. Eine Auswertung der erzielten Abtötungsrate erfolgt 3 und 5 Tage nach Applikation. Der Versuch wird bei ca. 21°C und etwa 60 % relativer Luftfeuchtigkeit durchgeführt.

Verbindungen gemäss den Beispielen 1.2.1., 1.2.2. und 1.2.3. zeigen gute Wirkung in diesem Test.

3.12. Wirkung gegen Tetranychus urticae (OP-sensibel)

Die Primärblätter von Phaseolus vulgaris-Pflanzen werden 24 Stunden vor dem Versuch auf akarizide Wirkung mit einem infestierten Blattstück aus einer Massenzucht von Tetranychus urticae (OP-sens.) belegt (Mischpopulation). Die Toleranz bezieht sich auf die Verträglichkeit gegenüber Diazinon.

Die so behandelten infestierten Pflanzen werden mit einer Versuchslösung in Emulsionsform enthaltend 400 ppm der zu prüfenden Verbindung bis zur Tropfnässe besprüht. Während des Versuchsverlaufs stehen die Pflanzen in Gewächshauskabinen bei ca. 25°C mit etwa 50 % rel. Luftfeuchtigkeit.

Nach 6 Tagen werden Imagines und Larven (alle beweglichen Stadien) sowie abgelegte Eier unter dem Binokular auf lebende und tote Individuen ausgewertet.

Verbindungen gemäss den Beispielen 1.2.1., 1.2.2. und 1.2.3. zeigen in diesem Versuch gute Wirkung.

3.13. Wirkung gegen Panonychus ulmi (OP- und Carbamat-resistent)

Eingetopfte Apfelsämlinge mit etwa 20-30 Blättern werden mit jeweils 60 adulten Weibchen von Panonychus ulmi besiedelt. Nach sieben Tagen werden die infestierten Pflanzen mit einer wässrigen Emulsion enthaltend 100 ppm der zu prüfenden Verbindung bis zur Tropfnässe besprüht. Die behandelten Pflanzen werden dann während 14 Tagen bei ca. 25°C und etwa 50 % relativer Luftfeuchtigkeit im Gewächshaus aufgestellt.

Nach dieser Zeit erfolgt die Auswertung, indem man 20 Blätter pro Pflanze entnimmt, die Milbenpopulation mittels einer Abbürst-Vorrichtung von den entnommenen Blättern entfernt und unter dem Binocular nach Eiern, postembryonalen Stadien und Adulten auszählt. Bewertet wird die prozentuale Reduktion der Milbenpopulation gegenüber unbehandelten Kontrollen.

Verbindungen gemäss den Beispielen 1.2.1., 1.2.2. und 1.2.3. zeigen gute Wirkung im obigen Test.

3.14. Wirkung gegen Dermanyssus gallinae

In einem nach oben offenen Glasbehälter werden 2 bis 3 ml einer 100 ppm Wirkstoff enthaltenden Lösung und ca. 200 Milben in unterschiedlichen Entwicklungsstadien gegeben. Anschliessend wird der Behälter mit einem Wattebausch verschlossen, 10 Minuten lang bis zur vollständigen Benetzung der Milben geschüttelt und dann kurzfristig umgekehrt, damit die restliche Testlösung von der Watte aufgenommen werden kann. Nach 3 Tagen wird die Mortalität der Milben durch Auszählen der toten Individuen ermittelt und in Prozenten angegeben.

Verbindungen gemäss den Beispielen 1.2.1., 1.2.2. und 1.2.3. zeigen 80-100%ige Wirkung (Mortalität).

## Patentansprüche

1. Verbindungen der Formel I

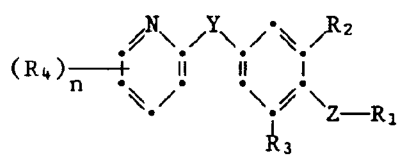 (I),

worin

$R_1$ für $C_1$-$C_{12}$-Alkyl, ein- oder mehrfach durch Halogen und/oder $C_1$-$C_6$-Alkoxy, substituiertes $C_1$-$C_{12}$-Alkyl, $C_3$-$C_8$-Cycloalkyl, ein- oder mehrfach durch $C_1$-$C_3$-Alkyl substituiertes $C_3$-$C_8$-Cycloalkyl oder $C_3$-$C_8$-Cycloalkyl-$C_1$-$C_4$-alkyl;

$R_2$ und $R_3$ je für $C_1$-$C_5$-Alkyl oder $C_5$-$C_6$-Cycloalkyl;

$R_4$ je für Wasserstoff, Halogen, $C_1$-$C_4$-Alkyl oder ein- oder mehrfach durch Halogen substituiertes $C_1$-$C_4$-Alkyl;

n für 1,2,3 oder 4;

Y für S, SO oder $SO_2$;

Z für -NH-CS-NH-, -N=C($SR_5$)-NH- oder -N=C=N-; und

$R_5$ für $C_1$-$C_{10}$-Alkyl oder Allyl

stehen, sowie ihre Salze mit organischen oder anorganischen Säuren.

2. Verbindungen der Formel I gemäss Anspruch 1, worin $R_1$ für $C_1$-$C_8$-Alkyl, ein- oder mehrfach durch Halogen und/oder $C_1$-$C_5$-Alkoxy substituiertes $C_1$-$C_8$-Alkyl oder $C_5$-$C_6$-Cycloalkyl; $R_2$ für $C_1$-$C_5$-Alkyl; $R_3$ für $C_1$-$C_5$-Alkyl oder Cyclopentyl; $R_4$ je für Wasserstoff oder Halogen; n für 1 oder 2; Y für S, SO oder $SO_2$; Z für -NH-CS-NH-, -N=C($SR_5$)-NH-oder -N=C=N-; und $R_5$ für $C_1$-$C_5$-Alkyl oder Allyl stehen.

3. Verbindungen der Formel I gemäss Anspruch 2, worin $R_1$ für $C_1$-$C_5$-Alkyl oder Cyclopentyl; $R_2$ und $R_3$ je für $C_1$-$C_3$-Alkyl; $R_4$ für Chlor; n für 1 oder 2; Y für S; und Z für -NH-CS-NH- stehen.

4. Verbindungen der Formel I gemäss Anspruch 2, worin $R_1$ für $C_1$-$C_5$-Alkyl oder Cyclopentyl; $R_2$ und $R_3$ je für $C_1$-$C_3$-Alkyl; $R_4$ für Chlor; n für 1 oder 2; Y für S; Z für -N=C($SR_5$)-NH-; und $R_5$ für $C_1$-$C_4$-Alkyl stehen.

5. Verbindungen der Formel I gemäss Anspruch 2, worin $R_1$ für $C_1$-$C_5$-Alkyl oder Cyclopentyl; $R_2$ und $R_3$ je für $C_1$-$C_3$-Alkyl; $R_4$ für Chlor; n für 1 oder 2; Y für S; und Z für -N=C=N- stehen.

6. Verbindung gemäss Anspruch 3 der Formeln

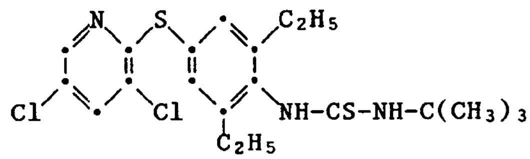

,

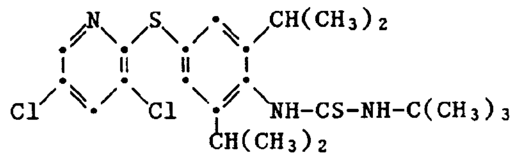

,

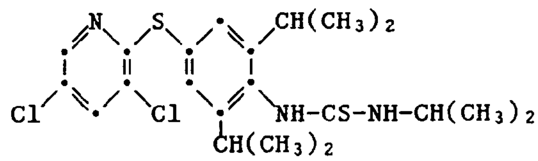

,

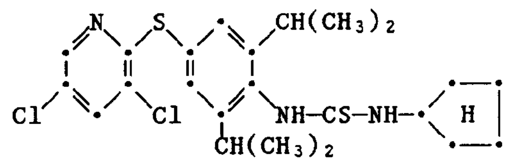

,

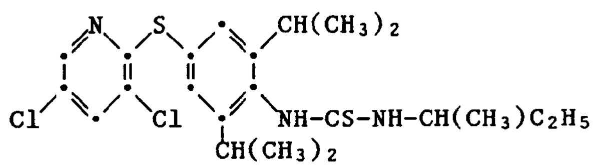

,

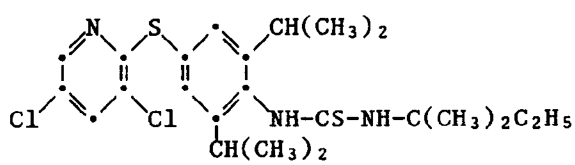

,

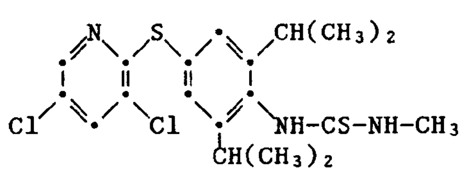

,

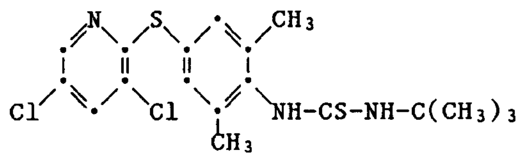 ,

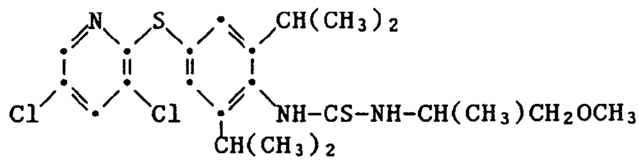 ,

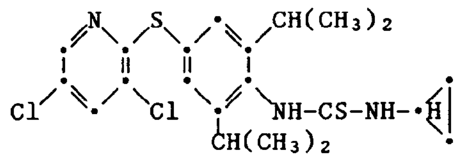 ,

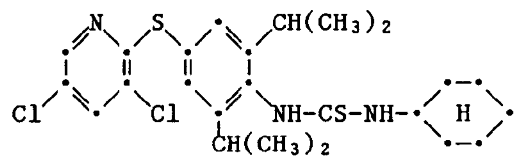 ,

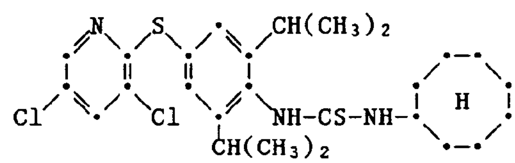 ,

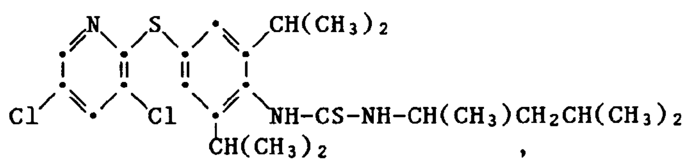 ,

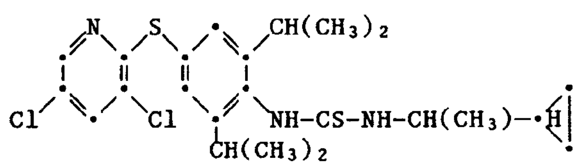 und

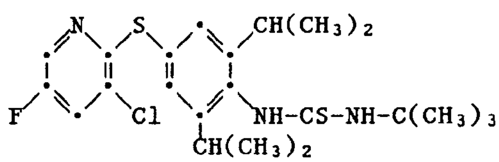 .

7. Verbindungen gemäss Anspruch 4 der Formeln

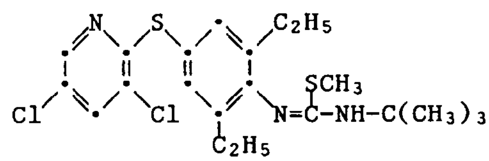

,

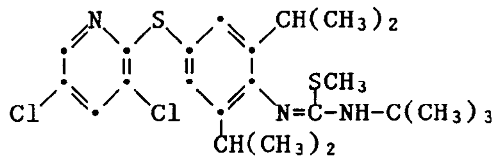

,

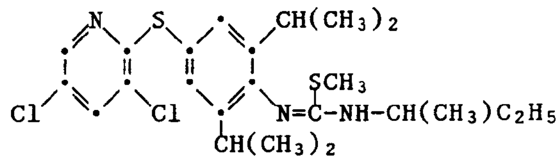

,

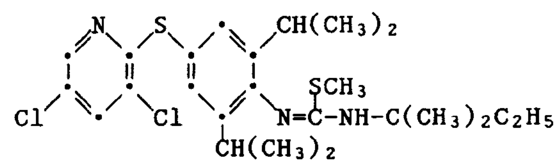

,

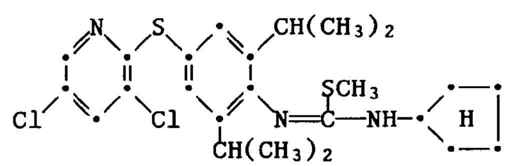

,

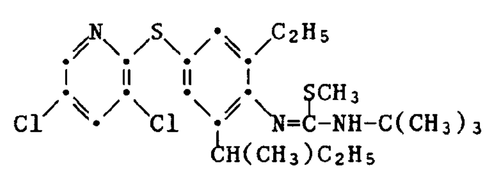

und

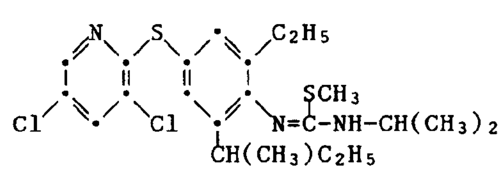

.

8. Verbindungen gemäss Anspruch 5 der Formeln

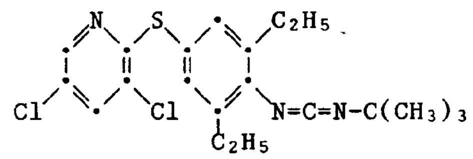
,

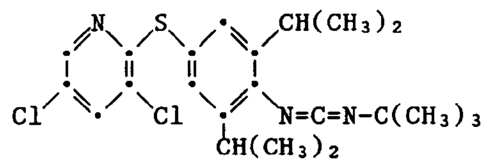
,

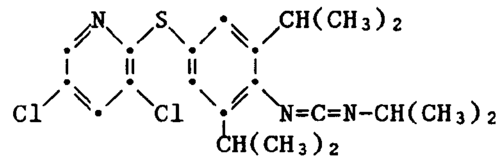
,

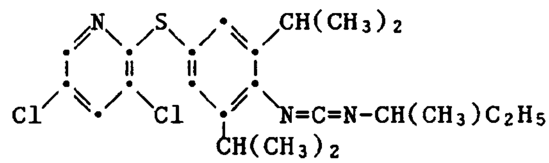
,

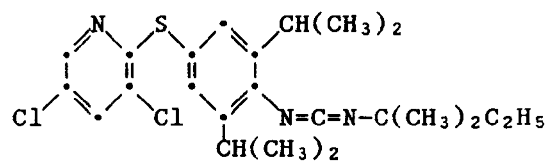
,

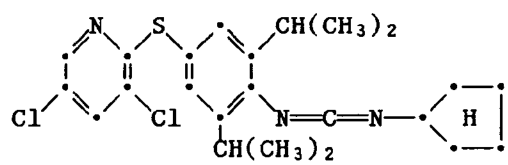
,

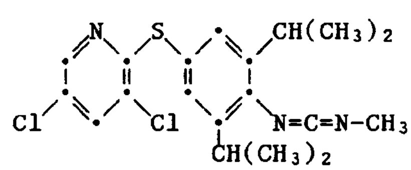
,

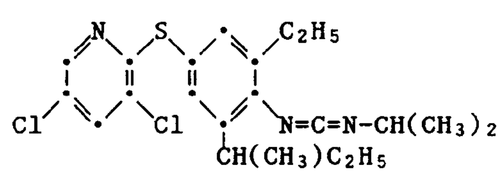
,

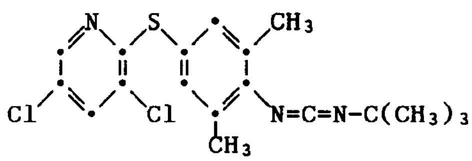
,

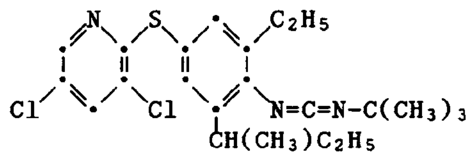
,

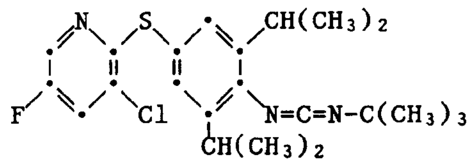
,

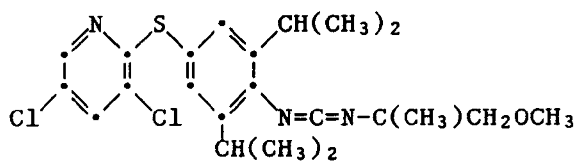
,

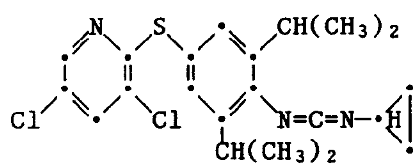
,

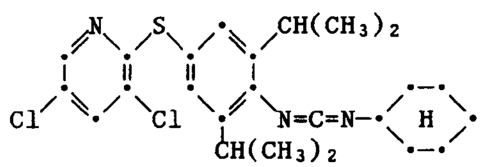
,

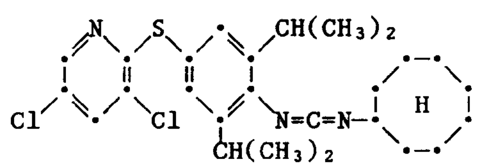
,

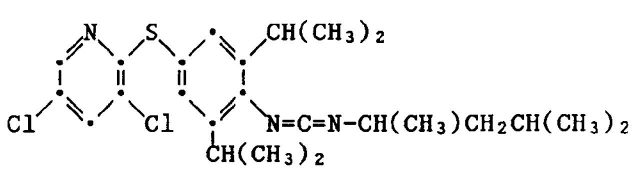
und

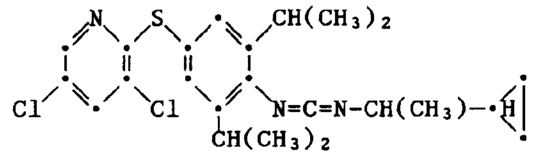

.

9. Verfahren zur Herstellung einer Verbindung der Formel I gemäss Anspruch 1, dadurch gekennzeichnet, dass man

A) ein Isothiocyanat der Formel II

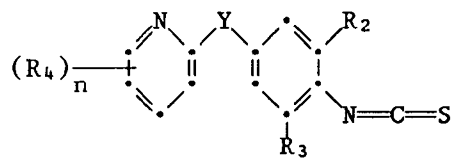

(II)

mit einem Amin der Formel III

$H_2N—R_1$ (III)

in einem organischen Lösungs- oder Verdünnungsmittel bei Normaldruck und einer Temperatur von 0 bis +150°C zum Thioharnstoff umsetzt und gegebenenfalls

B) den erhaltenen Thioharnstoff mit einer Verbindung der Formel IV

$X—R_5$ (IV)

in einem inerten organischen Lösungsmittel unter leicht erhöhtem oder normalem Druck und bei einer Temperatur von +10 bis 250°C zum Isothioharnstoff umsetzt oder

C) den erhaltenen Thioharnstoff durch Abspalten von Schwefelwasserstoff in einem aprotischen organischen Lösungs- oder Verdünnungsmittel unter Normaldruck und bei einer Temperatur von 0 bis +150°C in das Carbodiimid überführt, wobei in den Formeln II, III und IV $R_1$, $R_2$, $R_3$, $R_4$, $R_5$, Y und n die unter Formel I angegebenen Bedeutungen haben und X für eine Abgangsgruppe steht.

10. Schädlingsbekämpfungsmittel, welches als aktive Komponente mindestens eine Verbindung der Formel I gemäss Anspruch 1 oder eines ihrer Salze mit einer organischen oder anorganischen Säure zusammen mit geeigneten Trägern und/oder Zuschlagstoffen enthält.

11. Schädlingsbekämpfungsmittel gemäss Anspruch 10, welches als aktive Komponente mindestens eine Verbindung der Formel I gemäss Anspruch 2 enthält.

12. Schädlingsbekämpfungsmittel gemäss Anspruch 10, welches als aktive Komponente mindestens eine Verbindung der Formel I gemäss Anspruch 3 enthält.

13. Schädlingsbekämpfungsmittel gemäss Anspruch 10, welches als aktive Komponente mindestens eine Verbindung der Formel I gemäss Anspruch 4 enthält.

14. Schädlingsbekämpfungsmittel gemäss Anspruch 10, welches als aktive Komponente mindestens eine Verbindung der Formel I gemäss Anspruch 5 enthält.

15. Verwendung einer Verbindung der Formel I gemäss Anspruch 1 oder eines ihrer Salze mit einer organischen oder anorganischen Säure zur Bekämpfung von Insekten und Arachniden an Tieren und Pflanzen.

16. Verwendung gemäss Anspruch 15 einer Verbindung der Formel I zur Bekämpfung von pflanzenschädigenden Insekten und Arachniden.

17. Verbindungen der Formel V

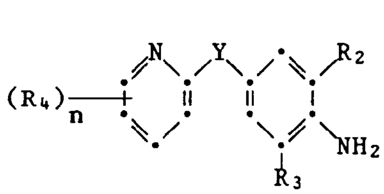

(V),

worin $R_2$ und $R_3$ je für $C_1$-$C_5$-Alkyl oder $C_5$-$C_6$-Cycloalkyl;

$R_4$ je für Wasserstoff, Halogen, $C_1$-$C_4$-Alkyl oder ein- oder mehrfach durch Halogen substituiertes $C_1$-$C_4$-Alkyl;

n für 1,2,3 oder 4; und

Y für S, SO oder $SO_2$
stehen.

18. Verbindungen gemäss Anspruch 17, worin $R_2$ für $C_1$-$C_5$-Alkyl; $R_3$ für $C_1$-$C_5$-Alkyl oder Cyclopentyl; $R_4$ je für Wasserstoff oder Halogen; n für 1 oder 2; und Y für S, SO oder $SO_2$ stehen.

19. Verbindungen gemäss Anspruch 18, worin $R_2$ und $R_3$ je für $C_1$-$C_3$-Alkyl; $R_4$ für Chlor; n für 1 oder 2; und Y für S stehen.

20. Die Verbindungen gemäss Anspruch 19 der Formeln

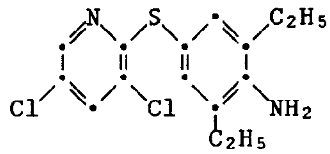
,

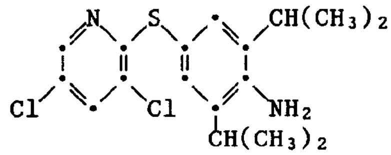
,

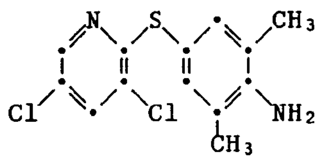
,

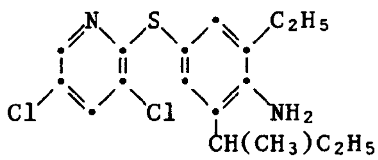
und

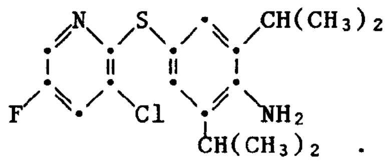
.

21. Verbindungen der Formel II

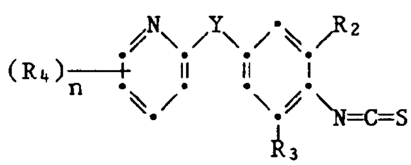
(II)

worin $R_2$ und $R_3$ je für $C_1$-$C_5$-Alkyl oder $C_5$-$C_6$-Cycloalkyl;

$R_4$ je für Wasserstoff, Halogen, $C_1$-$C_4$-Alkyl oder ein- oder mehrfach durch Halogen substituiertes $C_1$-$C_4$-Alkyl;

n für 1,2,3 oder 4; und

Y für S, SO oder $SO_2$

stehen.

22. Verbindungen gemäss Anspruch 21, worin $R_2$ für $C_1$-$C_5$-Alkyl; $R_3$ für $C_1$-$C_5$-Alkyl oder Cyclopentyl; $R_4$ je für Wasserstoff oder Halogen; n für 1 oder 2; und Y für S, SO oder $SO_2$ stehen.

23. Verbindungen gemäss Anspruch 22, worin $R_2$ und $R_3$ je für $C_1$-$C_3$-Alkyl; $R_4$ für Chlor; n für 1 oder 2; und Y für S stehen.

24. Die Verbindungen gemäss Anspruch 23 der Formeln

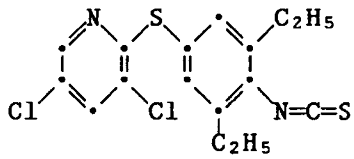

,

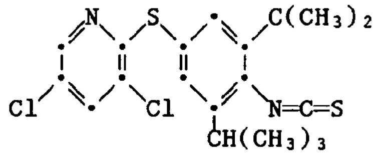

,

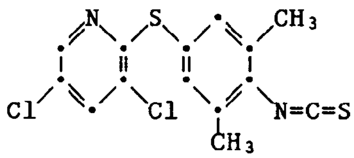

,

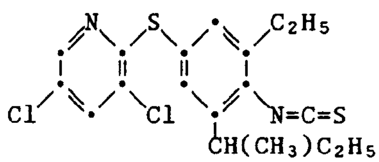

und

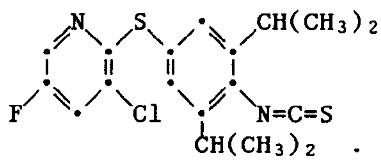

.

## Claims

1. A compound of formula I

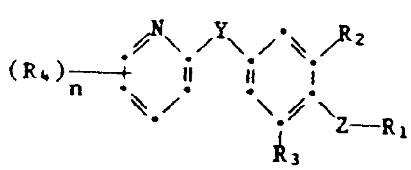

(I)

wherein

$R_1$ represents $C_1$-$C_{12}$alkyl, $C_1$-$C_{12}$alkyl mono- or poly-substituted by halogen and/or by $C_1$-$C_6$alkoxy, $C_3$-$C_8$cycloalkyl, $C_3$-$C_8$cycloalkyl mono- or poly-substituted by $C_1$-$C_3$alkyl, or $C_3$-$C_8$cycloalkyl-$C_1$-$C_4$alkyl;

$R_2$ and $R_3$ each represents $C_1$-$C_5$alkyl or $C_5$-$C_6$cycloalkyl;
each $R_4$ represents hydrogen, halogen, $C_1$-$C_4$alkyl, or $C_1$-$C_4$alkyl mono- or poly-substituted by halogen;
n represents 1, 2, 3 or 4;
Y represents S, SO or $SO_2$;
Z represents -NH-CS-NH-, -N=C($SR_5$)-NH- or -N=C=N-; and
$R_5$ represents $C_1$-$C_{10}$alkyl or allyl,

or a salt thereof with an organic or inorganic acid.

2. A compound of formula I according to claim 1 wherein $R_1$ represents $C_1$-$C_8$alkyl, $C_1$-$C_8$alkyl mono- or poly-substituted by halogen and/or by $C_1$-$C_5$alkoxy, or $C_5$-$C_6$cycloalkyl; $R_2$ represents $C_1$-$C_5$alkyl; $R_3$ represents $C_1$-$C_5$alkyl or cyclopentyl; each $R_4$ represents hydrogen or halogen; n represents 1 or 2; Y represents S, SO or $SO_2$; Z represents -NH-CS-NH-, -N=C($SR_5$)-NH- or -N=C=N-; and $R_5$ represents $C_1$-$C_5$alkyl or allyl.

3. A compound of formula I according to claim 2 wherein $R_1$ represents $C_1$-$C_5$alkyl or cyclopentyl; $R_2$ and $R_3$ each represents $C_1$-$C_3$alkyl; $R_4$ represents chlorine; n represents 1 or 2; Y represents S; and Z represents -NH-CS-NH-.

4. A compound of formula I according to claim 2 wherein $R_1$ represents $C_1$-$C_5$alkyl or cyclopentyl; $R_2$ and $R_3$ each represents $C_1$-$C_3$alkyl; $R_4$ represents chlorine; n represents 1 or 2; Y represents S; Z represents -N=C($SR_5$)-NH-; and $R_5$ represents $C_1$-$C_4$alkyl.

5. A compound of formula I according to claim 2 wherein $R_1$ represents $C_1$-$C_5$alkyl or cyclopentyl; $R_2$ and $R_3$ each represents $C_1$-$C_2$alkyl; $R_4$ represents chlorine; n represents 1 or 2; Y represents S; and Z represents -N=C=N-.

6. A compound according to claim 3 of the formula

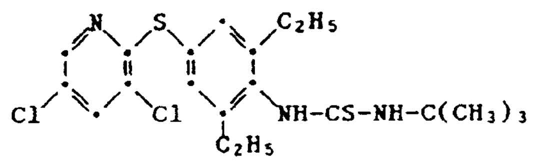

,

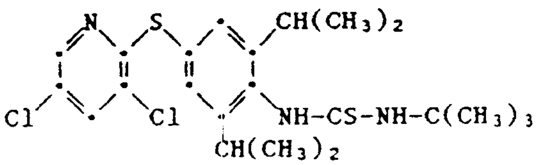

,

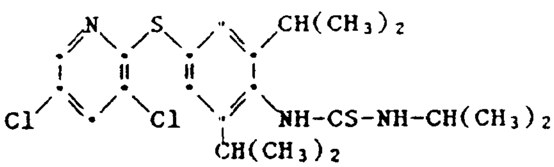

,

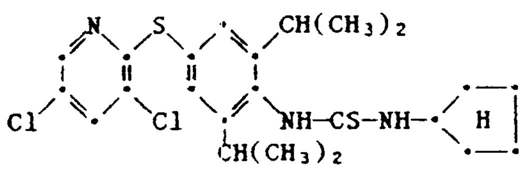

,

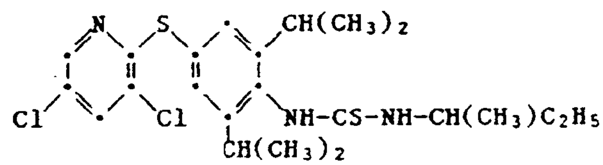

,

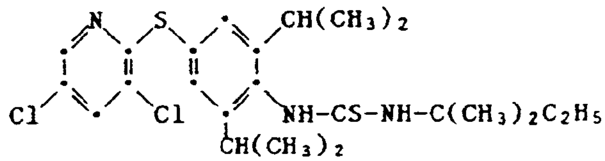

,

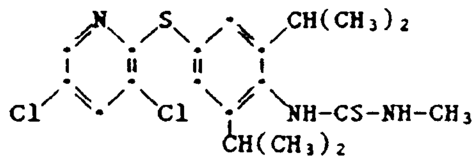

,

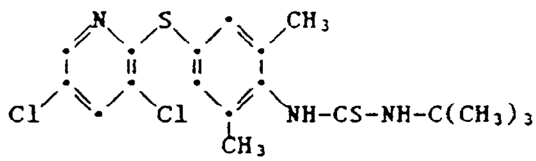

,

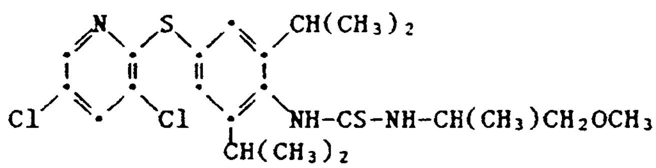

,

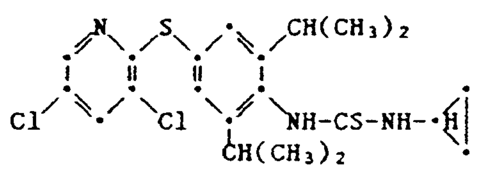

,

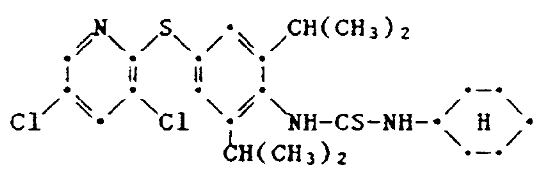

,

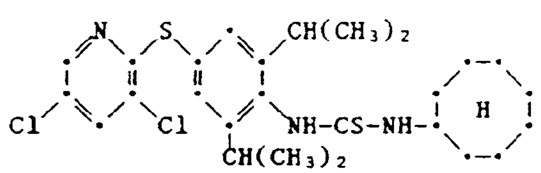

,

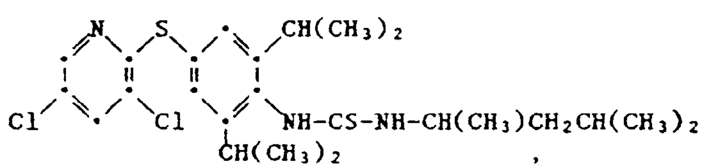

,

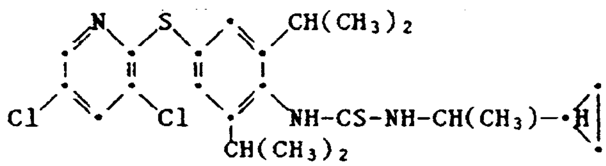

or

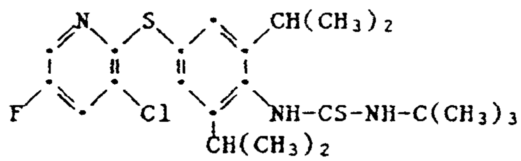

.

7. A compound according to claim 4 of the formula

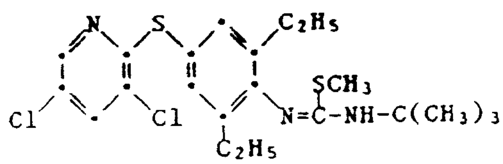

,

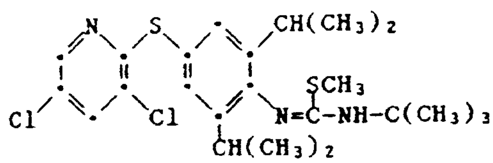

,

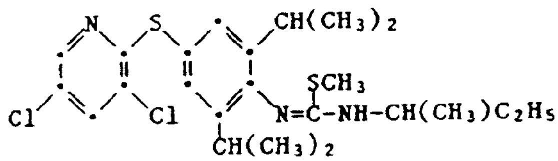

,

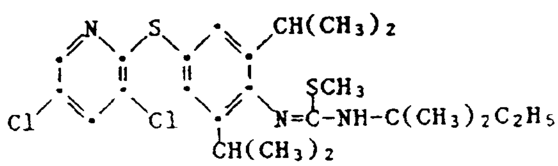

,

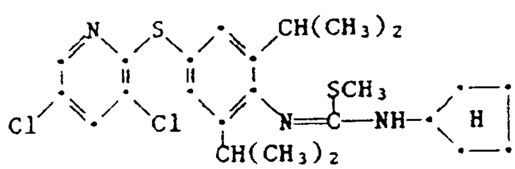

,

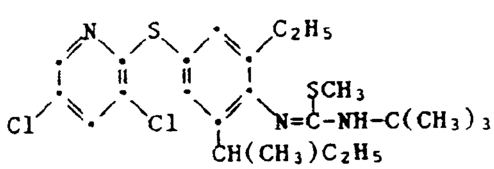

or

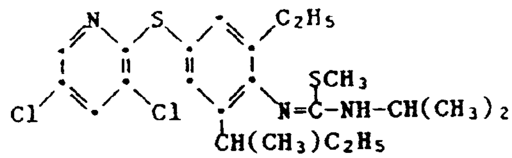

.

8. A compound according to claim 5 of the formula

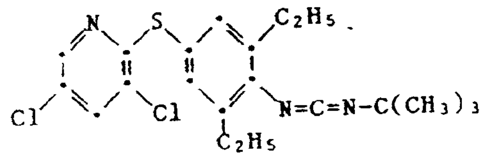

,

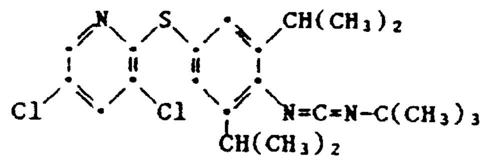

,

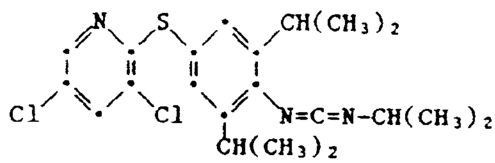

,

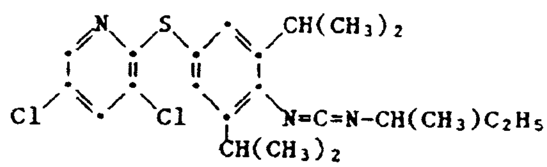

,

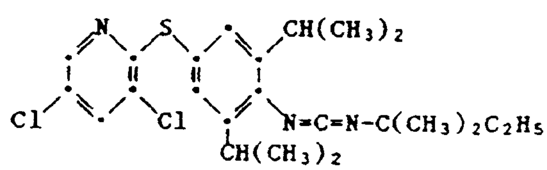

,

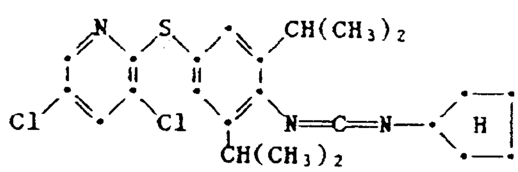

,

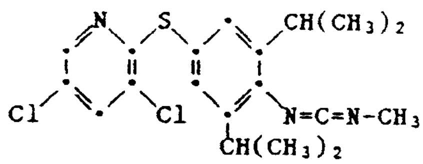
,

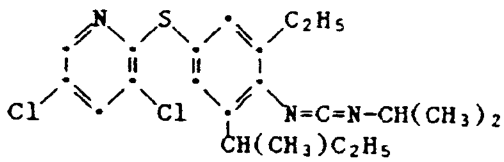
,

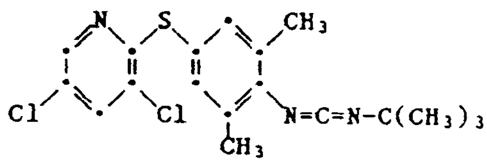
,

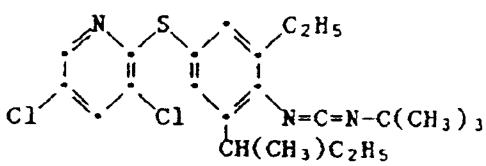
,

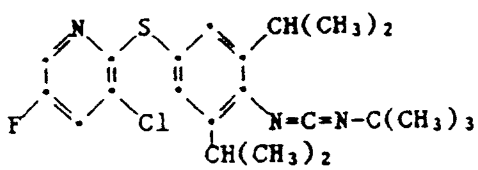
,

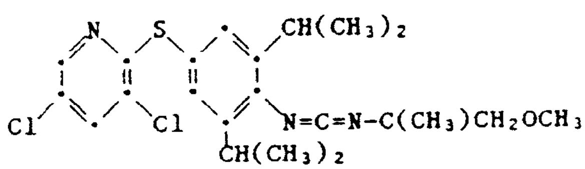
,

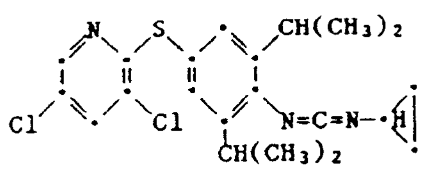
,

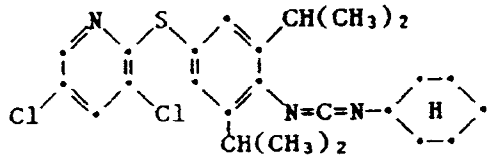

,

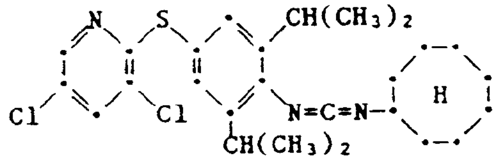

,

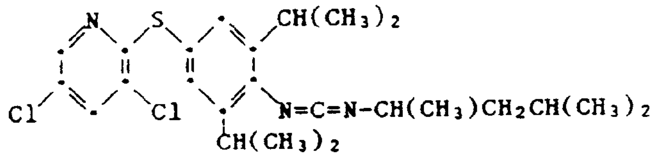

or

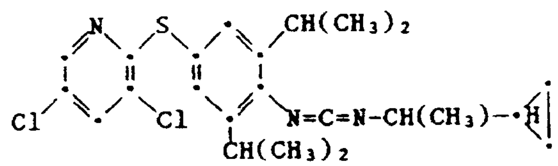

.

9. A process for the preparation of a compound of formula I according to claim 1, wherein
A) an isothiocyanate of formula II

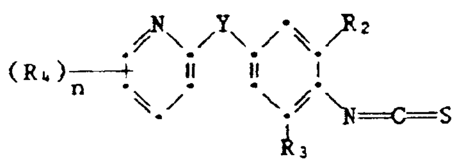

(II)

is reacted with an amine of formula III

$H_2N\text{-}R_1$ (III)

in an organic solvent or diluent, at normal pressure and at a temperature of from 0 to +150°C, to form thiourea and optionally
B) the resulting thiourea is reacted with a compound of formula IV

$X\text{-}R_5$ (IV)

in an inert organic solvent, at slightly elevated or normal pressure and at a temperature of from +10 to 250°C, to form isothiourea, or
C) the resulting thiourea is converted into the carbodiimide by removal of hydrogen sulfide in an aprotic organic solvent or diluent at normal pressure and at a temperature of from 0 to +150°C, wherein in formulae II, II and IV $R_1$, $R_2$, $R_3$, $R_4$, $R_5$, Y and n have the meanings given under formula I and X represents a leaving group.

10. A pesticidal composition which comprises as active ingredient at least one compound of formula I according to claim 1, or a salt thereof with an organic or inorganic acid, together with suitable carriers and/or adjuvants.

11. A pesticidal composition according to claim 10 which comprises as active ingredient at least one compound of formula I according to claim 2.

12. A pesticidal composition according to claim 10 which comprises as active ingredient at least one compound of formula I according to claim 3.

13. A pesticidal composition according to claim 10 which comprises as active ingredient at least one compound of formula I according to claim 4.

14. A pesticidal composition according to claim 10 which comprises as active ingredient at least one compound of formula I according to claim 5.

15. The use of a compound of formula I according to claim 1, or a salt thereof with an organic or inorganic acid, for controlling insects and arachnids in and on animals and plants.

16. The use according to claim 15 of a compound of formula I for controlling plant-destructive insects and arachnids.

17. A compound of formula V

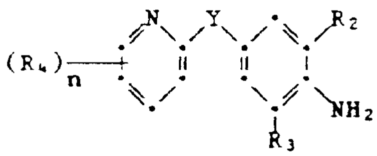

(V)

wherein $R_2$ and $R_3$ each represents $C_1$-$C_5$alkyl or $C_5$-$C_6$cycloalkyl;

each $R_4$ represents hydrogen, halogen, $C_1$-$C_4$alkyl, or $C_1$-$C_4$alkyl mono- or poly-substituted by halogen;

n represents 1, 2, 3 or 4; and

Y represents S, SO or $SO_2$.

18. A compound according to claim 17 wherein $R_2$ represents $C_1$-$C_5$alkyl; $R_3$ represents $C_1$-$C_5$alkyl or cyclopentyl; each $R_4$ represents hydrogen or halogen; n represents 1 or 2; and Y represents S, SO or $SO_2$.

19. A compound according to claim 18 wherein $R_2$ and $R_3$ each represents $C_1$-$C_3$alkyl; $R_4$ represents chlorine; n represents 1 or 2; and Y represents S.

20. A compound according to claim 19 of the formula

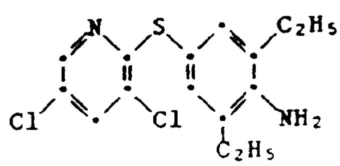

,

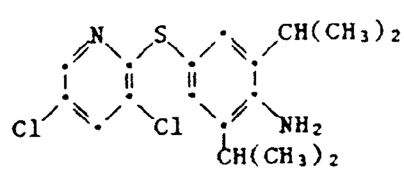

,

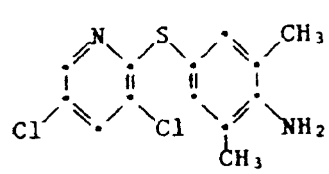

,

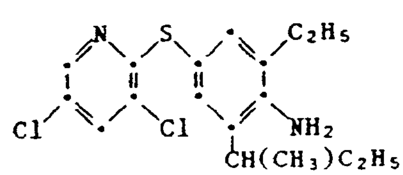

or

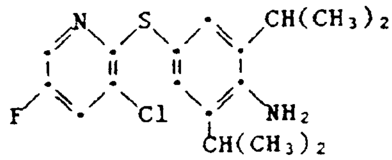

.

21. A compound of formula II

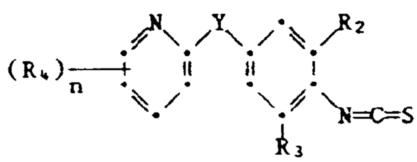

(II)

wherein $R_2$ and $R_3$ each represents $C_1$-$C_5$alkyl or $C_5$-$C_6$cycloalkyl;

each $R_4$ represents hydrogen, halogen, $C_1$-$C_4$alkyl, or $C_1$-$C_4$alkyl mono- or poly-substituted by halogen;

n represents 1, 2, 3 or 4; and

Y represents S, SO or $SO_2$.

22. A compound according to claim 21 wherein $R_2$ represents $C_1$-$C_5$alkyl; $R_3$ represents $C_1$-$C_5$alkyl or cyclopentyl; each $R_4$ represents hydrogen or halogen; n represents 1 or 2; and Y represents S, SO or $SO_2$.

23. A compound according to claim 22 wherein $R_2$ and $R_3$ each represents $C_1$-$C_3$alkyl; $R_4$ represents chlorine; n represents 1 or 2; and Y represents S.

24. A compound according to claim 23 of the formula

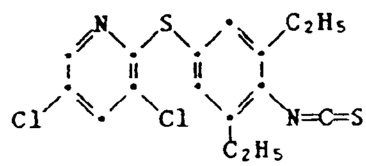

,

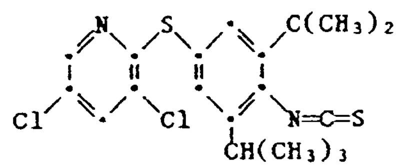

,

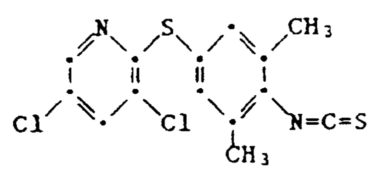

,

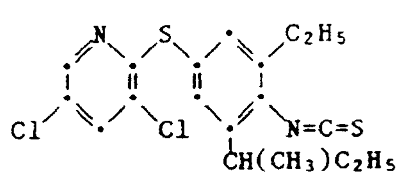

or

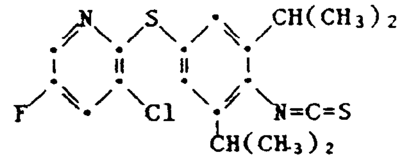

.

## Revendications

1. Composés de formule I

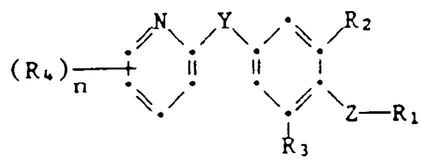

(I),

où

$R_1$ représente un alkyle en $C_1$-$C_{12}$, un alkyle en $C_1$-$C_{12}$ une ou plusieurs fois substitué par un halogène et/ou un alcoxy en $C_1$-$C_6$, un cycloalkyle en $C_3$-$C_8$, un cycloalkyle en $C_3$-$C_8$ ou un cycloalkyle en $C_3$-$C_8$ alkyle en $C_1$-$C_4$ une ou plusieurs fois substitué par un alkyle en $C_1$-$C_5$ ;

$R_2$ et $R_3$ représentent chacun un alkyle en $C_1$-$C_5$ ou un cycloalkyle en $C_5$-$C_6$ ;

$R_4$ représente l'hydrogène, un halogène, un alkyle en $C_1$-$C_4$ ou un alkyle en $C_1$-$C_4$ une ou plusieurs fois substitué par un halogène ;

n représente 1, 2, 3 ou 4 ;

Y représente S, SO ou $SO_2$ ;

Z représente -NH-CS-NH-, -N=C($SR_5$)-NH- ou -N=C=N- et

$R_5$ représente un alkyle en $C_1$-$C_{10}$ ou un allyle,

ainsi que leurs sels avec les acides organiques ou minéraux.

2. Composés de formule I selon la revendication 1, où $R_1$ représente un alkyle en $C_1$-$C_8$, un alkyle en $C_1$-$C_8$ ou un cycloalkyle en $C_5$-$C_6$ une ou plusieurs fois substitué par un halogène et/ou par un alcoxy en $C_1$-$C_5$ ; $R_2$ représente un alkyle en $C_1$-$C_5$ ; $R_3$ représente un alkyle en $C_1$-$C_5$ ou le cyclopentyle ; $R_4$ représente l'hydrogène ou un halogène ; n représente 1 ou 2 ; Y représente S, SO ou $SO_2$ ; Z représente -NH-CS-NH-, -N=C($SR_5$)-NH- ou -N=C=N- ; et $R_5$ représente un alkyle en $C_1$-$C_5$ ou l'allyle.

3. Composés de formule I selon la revendication 2, où $R_1$ représente un alkyle en $C_1$-$C_5$ ou le cyclopentyle; $R_2$ et $R_3$ représentent chacun un alkyle en $C_1$-$C_3$ ; $R_4$ représente le chlore ; n représente 1 ou 2 ; Y représente S ; et Z représente -NH-CS-NH-.

4. Composés de formule I selon la revendication 2, où $R_1$ représente un alkyle en $C_1$-$C_5$ ou le cyclopentyle; $R_2$ et $R_3$ représentent chacun un alkyle en $C_1$-$C_3$ ; $R_4$ représente le chlore ; n représente 1 ou 2 ; Y représente S ; Z représente -N=C($SR_5$)-NH- ; et $R_5$ représente un alkyle en $C_1$-$C_4$.

5. Composés de formule I selon la revendication 2, où $R_1$ représente un alkyle en $C_1$-$C_5$ ou le cyclopentyle; $R_2$ et $R_3$ représentent chacun un alkyle en $C_1$-$C_3$ ; $R_4$ représente le chlore ; n représente 1 ou 2 ; Y représente S ; et Z représente -N=C=N-.

6. Composés selon la revendication 3 de formules

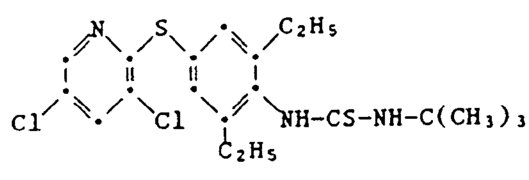

,

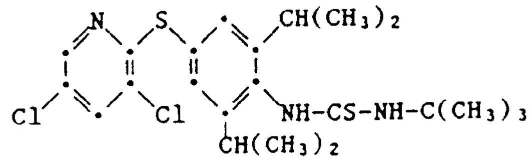
,

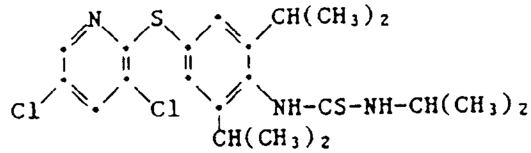
,

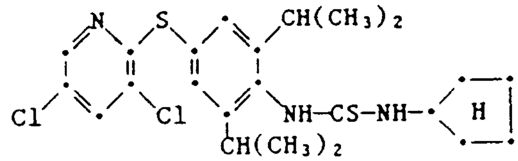
,

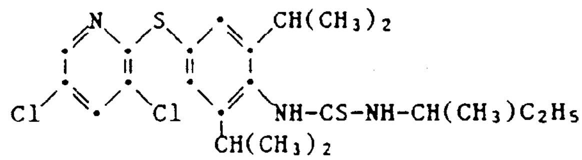
,

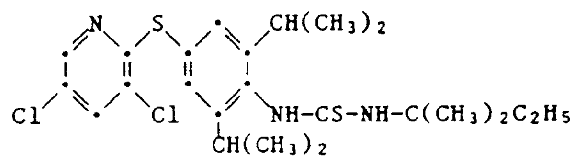
,

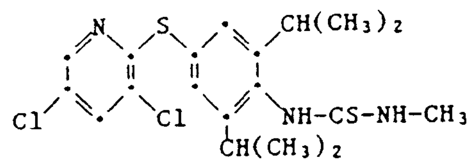
,

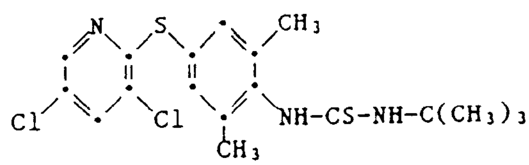
,

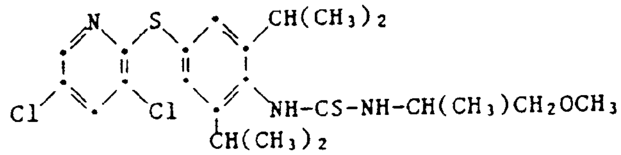

,

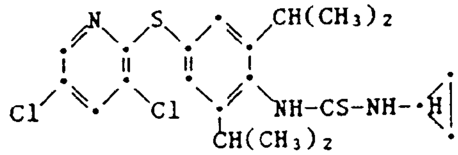

,

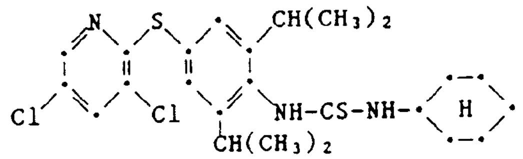

,

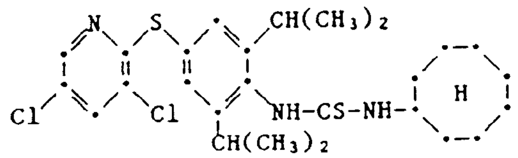

,

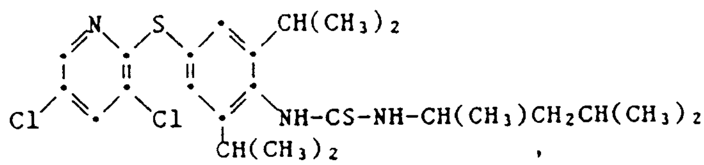

,

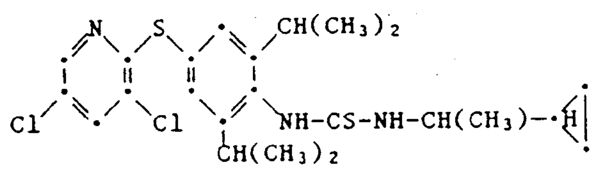

et

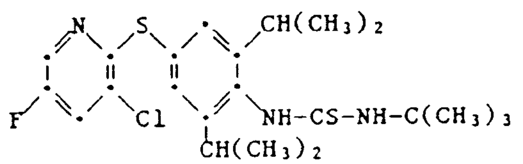

.

7. Composés selon la revendication 4, de formules

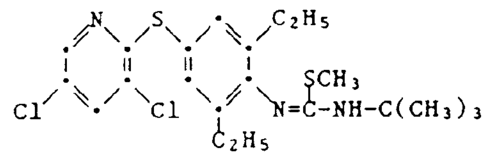

,

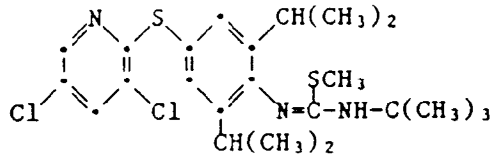

,

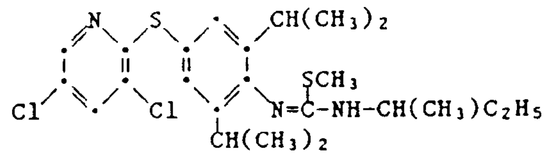

,

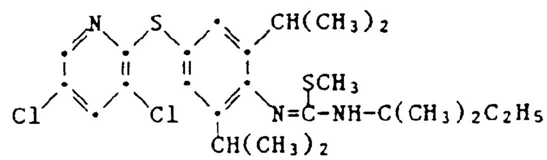

,

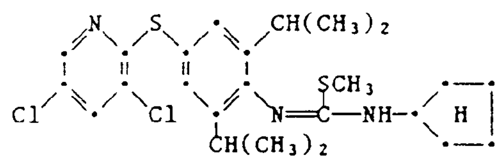

,

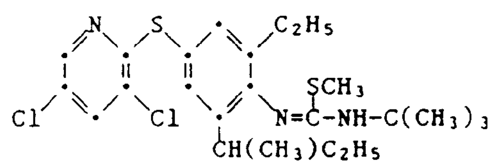

et

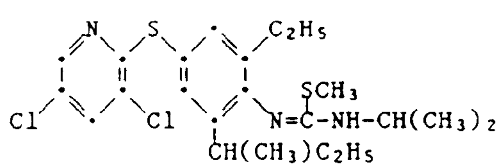

.

8. Composés selon la revendication 5, de formules

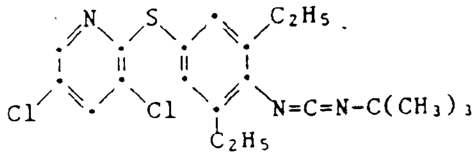

,

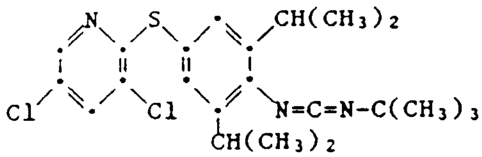

,

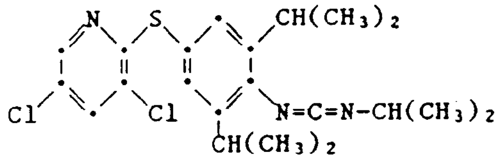

,

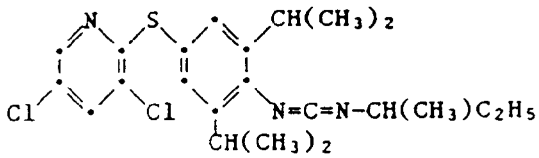

,

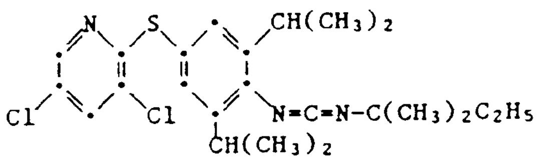

,

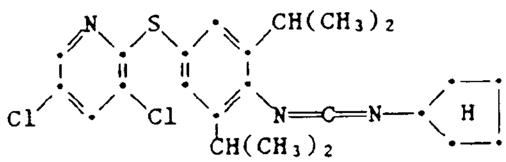

,

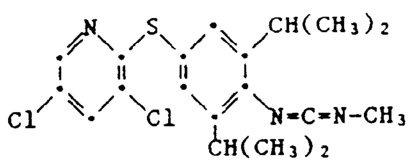

,

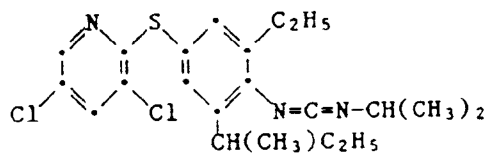
,

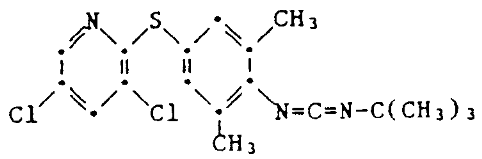
,

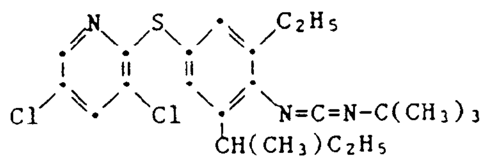
,

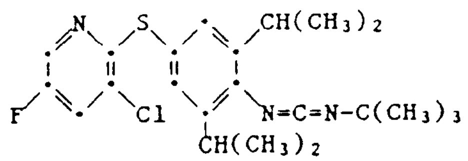
,

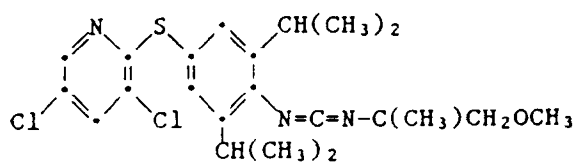
,

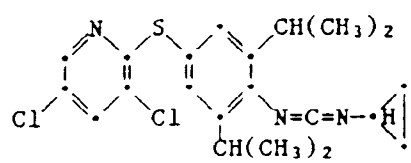
,

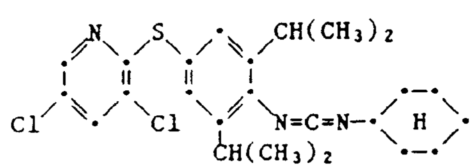
,

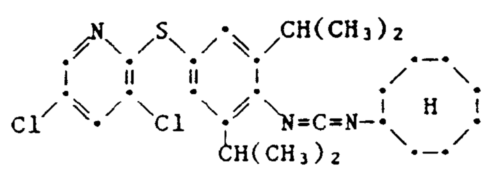
,

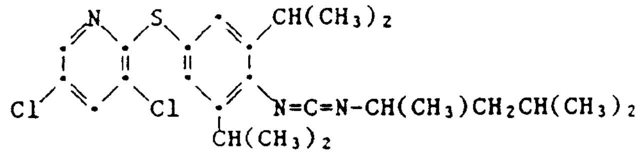 et

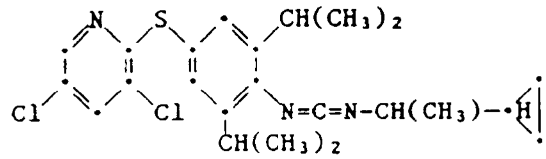 .

9. Procédé pour la préparation d'un composé de formule I selon la revendication 1, caractérisé

A) en ce que l'on fait réagir un isothiocyanate de formule II

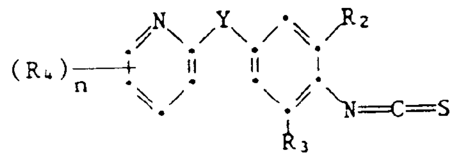 (II)

avec une amine de formule III

$$H_2N\text{-}R_1 \quad (III)$$

dans un solvant ou diluant organique à la pression normale et à une température allant de 0 à +150°C pour former une thiourée et éventuellement

B) en ce que l'on fait réagir la thiourée obtenue avec un composé de formule IV

$$X\text{—}R_5 \quad (IV)$$

dans un solvant organique inerte sous une pression légèrement augmentée ou à la pression normale et à une température allant de +10 à 250°C pour former une isothiourée ou

C) en ce que l'on transforme la thiourée obtenue en carbodiimide par clivage de l'acide sulfhydrique dans un solvant ou diluant organique aprotique à la pression normale et à une température allant de 0 à +150°C, $R_1$, $R_2$, $R_3$, $R_4$, $R_5$, Y et n ayant dans les formules II, III et IV, les significations indiquées sous la formule I et X représentant un groupe partant.

10. Agent de lutte contre les parasites contenant, comme composant actif, au moins un composé de formule I selon la revendication 1 ou l'un de ses sels avec un acide organique ou minéral, associé à des supports et/ou additifs appropriés.

11. Agent de lutte contre les parasites selon la revendication 10, contenant, comme composant actif, au moins un composé de formule I selon la revendication 2.

12. Agent de lutte contre les parasites selon la revendication 10, contenant, comme composant actif, un composé de formule I selon la revendication 3.

13. Agent de lutte contre les parasites selon la revendication 10, contenant, comme composant actif, au moins un composé de formule I selon la revendication 4.

14. Agent de lutte contre les parasites selon la revendication 10, contenant, comme composant actif, au moins un composé de formule I selon la revendication 5.

15. Utilisation d'un composé de formule I selon la revendication 1 ou de l'un de ses sels avec un acide organique ou minéral pour lutter contre les insectes et les arachnides sur les animaux et les plantes.

16. Utilisation d'un composé de formule I selon la revendication 15, pour lutter contre les insectes et arachnides nuisibles aux plantes.

17. Composés de formule V

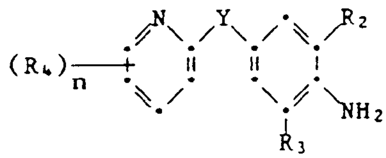 (V),

où $R_2$ et $R_3$ représentent chacun un alkyle en $C_1$-$C_5$ ou un cycloalkyle en $C_5$-$C_6$ ;

$R_4$ représente l'hydrogène, un halogène, un alkyle en $C_1$-$C_4$ ou un alkyle en $C_1$-$C_4$ une ou plusieurs fois substitué par un halogène ;

n représente 1, 2, 3 ou 4 ; et

Y représente S, SO ou $SO_2$.

18. Composés selon la revendication 17, où $R_2$ représente un alkyle en $C_1$-$C_5$ ; $R_3$ représente un alkyle en $C_1$-$C_5$ ou le cyclopentyle ; $R_4$ représente l'hydrogène ou un halogène ; n représente 1 ou 2 ; et Y représente S, SO ou $SO_2$.

19. Composés selon la revendication 18, où $R_2$ et $R_3$ représentent chacun un alkyle en $C_1$-$C_3$ ; $R_4$ représente le chlore ; n représente 1 ou 2 et Y représente S.

20. Les composés selon la revendication 19 de formules

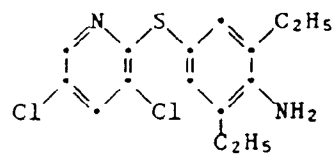 ,

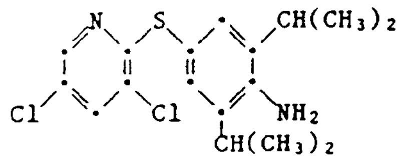 ,

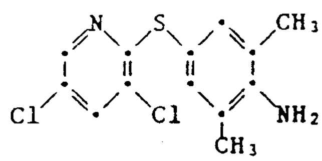 ,

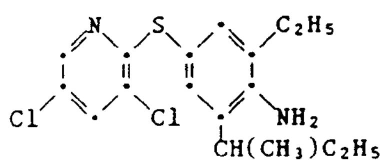 et

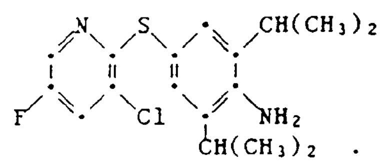 .

21. Composés de formule II

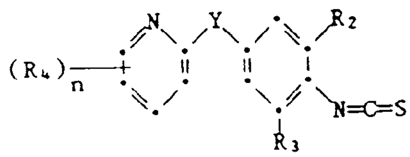

(II)

où

$R_2$ et $R_3$ représentent chacun un alkyle en $C_1$-$C_5$ ou un cycloalkyle en $C_5$-$C_6$ ;

$R_4$ représente l'hydrogène, un halogène, un alkyle en $C_1$-$C_4$ ou un alkyle en $C_1$-$C_4$ une ou plusieurs fois substitué par un halogène ;

n représente 1, 2, 3 ou 4 ; et

Y représente S, SO ou $SO_2$.

22. Composés selon la revendication 21, où $R_2$ représente un alkyle en $C_1$-$C_5$ ; $R_3$ représente un alkyle en $C_1$-$C_5$ ou le cyclopentyle ; $R_4$ représente l'hydrogène ou un halogène ; n représente 1 ou 2 ; et Y représente S, SO ou $SO_2$.

23. Composés selon la revendication 22, où $R_2$ et $R_3$ représentent chacun un alkyle en $C_1$-$C_3$ ; $R_4$ représente le chlore ; n représente 1 ou 2 ; et Y représente S.

24. Les composés selon la revendication 23 de formules

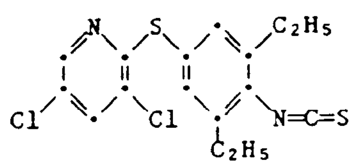

,

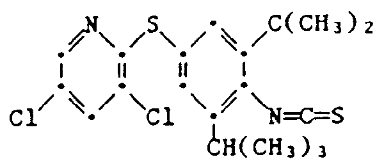

,

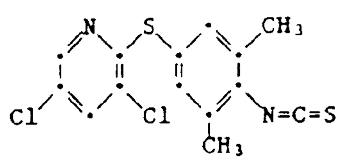

,

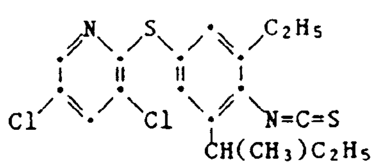

et

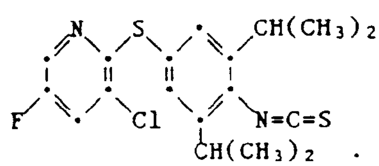

.